# EUROPÄISCHE PATENTSCHRIFT

(11) **EP 2 522 350 B1**
(45) Veröffentlichungstag und Bekanntmachung des Hinweises auf die Patenterteilung: **31.07.2013**
(21) Anmeldenummer: 11165926.4
(22) Anmeldetag: 12.05.2011
(51) Int. Cl.: A61K 31/498, A61K 31/575, A61P 39/06, A61P 37/04

(54) **Verwendung der Wirkstoffkombination aus einem 1-Diethylaminoethyl-3-Chinoxalin-2-on Derivat und einem Oxysterol zur Durchbrechung von Resistenzen bei der Behandlung von Krebs und zur Erhöhung der Immunleistungsfähigkeit bei Krebs, bakteriellen und viralen Erkrankungen, Autoimmunerkrankungen, erhöhter Stress- und Umweltbelastung**
Use of the active ingredient combination of a 1-diethylaminoethyl-3-chinoxalin-2-on derivative and an oxysterol for breaking resistance in the treatment of cancer and strengthening the immune system against cancer, bacterial and viral diseases, autoimmune diseases, increased stress and environmental influences
Utilisation de la combinaison de matière active constituée d'un dérivé de 1-diéthylaminoéthyle-3-chinoxaline-2-one et d'un oxystérol pour la rupture des résistances dans des traitements du cancer et pour l'augmentation de la capacité immunitaire lors de cancers, de maladies bactériennes ou virales, de maladies auto-immunes, d'effets de stress ou de pollution environnementale

(43) Veröffentlichungstag der Anmeldung: 14.11.2012
(73) Patentinhaber: Immunopharm AG, 9496 Balzers (LI)
(72) Erfinder: Knorr, Sylvia, 71732 Tamm (DE); Grimm, Christine, 78262 Gailingen (DE)
(74) Vertreter: Schüssler, Andrea

(56) Entgegenhaltungen:
- WO-A1-99/66931
- WO-A2-2005/105098
- DE-A1- 2 708 666
- GB-A- 2 053 924

## Beschreibung

### Gebiet der Erfindung

Die vorliegende Erfindung betrifft die Verwendung der Wirkstoffkombination aus 1-(2-Diethylaminoethyl)-3-(*p*-methoxy-benzyl)-1,2-dihydrochinoxalin-2-on und einem 7-β-Hydroxycholersterol zur Erhöhung der Immunleistungsfähigkeit bei Krebs, bakteriellen und viralen Erkrankungen, Autoimmunerkrankungen, erhöhter Stress- und Umweltbelastung.

### Stand der Technik

Ein gesundes und kräftiges Immunsystem kann Menschen dabei helfen, diverse Krankheitserreger zu bekämpfen und manchmal damit auch einen Krankheitsausbruch zu verhindern oder Krankheitssymptome zu mildem, beziehungsweise den Krankheitsverlauf zu verkürzen. Es verfügt über (a) mechanische Barrieren, die ein Eindringen der Schädlinge verhindern sollen; (b) zelluläre Bestandteile, die in den Blutgefäßen und Lymphbahnen zirkulieren und durch Aufnahme und Verdauung (Phagyozytose) den Erreger selbst vernichten oder durch die Produktion von Immunmodulatoren und Zytokinen die Immunreaktion des Organismus steuern und andere Abwehrzellen zum Ort der Entzündung locken; (c) humorale Bestandteile (Plasmaproteine), die passiv im Blut, bzw. der Lymph- und Gewebsflüssigkeit zirkulieren und als Botenstoffe oder zur Abwehr von Krankheitserregern dienen, wobei sie nicht in der Lage sind, aktiv an den Ort einer Infektion zu wandern und über (d) psychische Immunfaktoren. Verschiedene Faktoren können dazu führen, dass die Immunleistungsfähigkeit absinkt. Dazu zählen unter anderem eine gesundheitliche Beeinträchtigung durch chronische Erkrankungen, beispielsweise Krebs, bakterielle und virale Erkrankungen, Autoimmunerkrankungen oder erhöhte Stress- und Umweltbelastung, insbesondere infolge beruflicher Überlastung oder übermäßigem Leistungssport.

Gerade bei chronischen Erkrankungen und starken Stressbelastungen ist es notwendig, die Immunleistungsfähigkeit zu erhöhen. Als geeignete Maßnahmen gelten unter anderem ein gesunder Lebensstil, eine zusätzliche Substitution mit Mikronährstoffen, essentiellen Aminosäuren und Fettsäuren, die auch individuell nach Therapieplan erfolgen kann und eine ausreichende tiefenpsychologische Betreuung der Patienten.

In dem Patent US 4,281,120 ist erwähnt, dass 7H-1,3,4-Thiadiazolo-[3,2-a]-pyrimidin-7-on-5-carbonsäure-Verbindungen eine erhöhte Aktivität beim Stimulieren des Immunsystems haben. Daher können sie insbesondere bei der Behandlung von den Krankheiten verwendet werden, bei denen eine Erhöhung der Immunleistungsfähigkeit benötigt wird, beispielsweise bei bakteriellen und viralen Erkrankungen.

Die DE 35 208 46 A1 beschreibt die Verwendung des bekannten 8,8'-[Carbonylbis[imino-3,1-phenylencarbonylimino(4-methyl-3,1-phenylen)carbonylimino]]bis-1,3,5-naphthalin-trisulfonsaures-hexa-Natriumsalz (Suramin-Natrium) zur Verwendung als Immunstimulans bzw. zur Erhöhung der Immunleistungsfähigkeit bei der therapeutischen Behandlung des menschlichen oder tierischen Körpers. Suramin-Natrium zeigt eine ausgeprägte abwehrsteigemde Wirkung auf. Es zeigte sich, dass das Suramin-Natrium die Antikörpersynthese des Immunsystems Antigen-spezifisch steigert und die T-Lymphozytenproliferation erhöht.

Die EP 1 955 713 A1 betrifft Lipopetid-Konjugate, bei denen ein doppelt fettsäuresubstituiertes Cystein über die Carboxylgruppe an einen gut wasserlöslichen und physiologisch verträglichen sowie nicht immunogenen polymeren Konjugatrest gebunden ist, d.h. Biacyloxypropylcystein-Konjugate, die eine sehr gute makrophagenstimulierende Wirkung zeigen. Sie bieten sich insbesondere für zahlreiche Verwendungen an, beispielsweise zur Stimulierung der Antikörpersynthese, zur Infektionsabwehr, zur Immunstimulans, insbesondere gegenüber Tumoren.

Dr. Nikolaus P. Weger (DE 41 08 951 A1) hat festgestellt, dass Extrakte aus Eleutherokokkus, Milz und/oder Thymus zur Steigerung der körpereigenen Immunabwehr verwendet werden können, die bis dato lediglich als einzelne Substanzen bei verschiedenen Krebsarten, Leukämien oder bei AIDS eingesetzt wurden, aber ohne deutliche, messbare Heilerfolge. Erst durch die Kombination können die auf verschiedenen Wegen wirkenden Substanzen sich gegenseitig in der Steigerung der körpereigenen Immunabwehr zu einer kumulativen oder gar potenzierenden Steigerung der Wirkung unterstützen und zu einer deutlich messbaren Wirksamkeit führen. Beispielsweise werden durch die Kombination die für den Aufbau von Immunglobulinen erforderlichen Bausteine, wie z.B. Glycopeptide, zugeführt und zwar direkt als große Bausteine über die M-Zellen des Dünndarms. Ferner aktivieren die Thymusextrakte die Immunabwehr über die T-Zell-Reihe und deren Interleukine. Bei AIDS-Patienten zeigt sich die Wirkung über die Erhöhung der körpereigenen Immunglobuline, einen signifikanten Anstieg der körpereigenen Interferone, einer deutliche Zunahme des allgemeinen körperlichen Wohlbefindens, einer deutlichen Zunahme des reduzierten Körpergewichtes und einen deutlichen Rückgang bzw. Heilung von opportunistischen Erkrankungen. Ferner kann gemäß DE 42 19 745 A1 das kombinierte Produkt erweitert durch Magnesium-Aspartat, Kalium-Aspartat, Vitamin C gelöst in schwarzem Johannisbeersaft zur Substitution von dringend benötigten Bio-Elementen und Vitamin C als Antioxidans verwendet werden kann. Antioxidantien sind Radikalfänger und neutralisieren die Wirkung der Radikale. In dieser Kombination steigert das Immunstimulans insbesondere die geistige und körperliche Leistungsfähigkeit von Leistungssportlern. Bei älteren Menschen kann neben der körperlichen und geistigen Leistungssteigerung aus alters- und krankheitsbedingten Leiden vorgebeugt und somit länger ein lebenswertes Leben ermöglicht werden.

Das Patent US 5,563,140 beschreibt die Verwendung von 1-(Aminoalkyl)-3-benzyl-chinoxalin-2-on-Derivaten zur Herstellung pharmazeutischer Zusammensetzungen mit neuroprotektiver Wirkung, die auf die Fähigkeit, Glutamatrezeptoren in der exzitatorischen Neurotransmission im zentralen Nervensystem selektiv und reversibel als Antagonisten zu hemmen, zurückgeführt wird. Somit können diese pharmazeutischen Zusammensetzungen, für die Behandlung Glutamat-induzierter und Glutamat-Rezeptor vermittelter neurotoxischer Störungen verwendet werden, wie beispielsweise Funktionsstörungen vom Innenohr, z.B. Tinnitus und Hörschaden, und von der Retina, (post)traumatischer Läsionen und degenerativer Verfahren von Neuronen im zentralen Nervensystem, wie beispielsweise Alzheimer- und Parkinson-Krankheit. Beschrieben ist im US Patent 5,563,140 insbesondere die Verabreichung von 1-(2-Diethylaminoethyl)-3-(*p*-methoxy-benzyl)-1,2-dihydrochinoxalin-2-on mit dem internationalen Freinamen "Caroverin" (im Nachfolgenden "Caroverin" genannt). Die Wirkung von Caroverin wird seiner Fähigkeit zugeschrieben, Calciumkanäle zu blockieren, so dass die Calcium-vermittelte Aktivierung von myofibrillärer ATPase insbesondere in der glatten Muskulatur gehemmt wird.

Es ist desweiteren bekannt, dass mit 1-(Aminoalkyl)-3-chinoxalin-2-on Derivaten, insbesondere 1-Diethylaminoethyl-3-chinoxalin-2-on Derivaten, auch andere Krankheiten behandelt werden können, welche durch freie Radikale des Sauerstoffmetabolismus verursacht werden. Gemäß den im Patent US 6,573,265 gezeigten Untersuchungen sind die 1-Diethylaminoethyl-3-chinoxalin-2-on Derivate für die Behandlung von Schizophrenie, Lähmung der Gesichtsnerven, Redestörung infolge eines Schlaganfalls, und eines Hörsturzes geeignet.

Ein synergistischer Effekt bei der Behandlung von Krebs ist in der WO 2005/105098 A2 beschrieben, wobei durch die Kombination von Diethylaminoethyl-3-chinoxalin-2-on Derivaten und zytotoxischen Krebsmitteln mit direkt apoptotischer Wirkung ein effizientes und gut verträgliches Kombinationspräparat geschaffen worden ist, welches zu einer Rückbildung der Tumore führt. Überraschenderweise war das Präparat auch gegen krebsartige Plattenepithelzellen wirksam, deren Bekämpfung als sehr schwierig gilt. Die Studien haben gezeigt, dass die Konzentration des zytotoxischen Krebsmittels überraschenderweise verringert werden kann, wenn es zusammen mit 1-Diethylaminoethyl-3-chinoxalin-2-on Derivaten, vorzugsweise Caroverin oder dessen Derivaten, eingesetzt wird.

Im US Patent 6,573,265 ist die Verabreichung von Caroverin in Verbindung mit einer herkömmlichen Chemotherapie unter Verwendung der Präparate Irinothecan (antineoplastisches Mittel) und Oxaliplatin zur Behandlung von Dickdarm-, Hirn- und Rachentumoren beschrieben.

Es ist auch bekannt, dass 1-Diethylaminoethyl-3-chinoxalin-2-on Derivate aktive Verbindungen sind, welche einen Einfluss auf den Zu- und Abfluss von Antikrebsmitteln in und aus Zellen kontrollieren (Derwent Abstract, Publikation Nr. AN 83-45789 K). Durch die Zugabe der 1-Diethylaminoethyl-3-chinoxalin-2-on Derivate kann eine hohe Konzentration der Krebsmittel in den Zellen für eine längere Zeit erhalten bleiben. Allerdings wurde den 1-Diethylaminoethyl-3-chinoxalin-2-on Derivaten selbst keine eigentliche krebshemmende Wirkung zugeschrieben wird.

Tsuruo et al. (Cancer Research 42, 4730-4733, 1982) zeigen, dass einige Calcium-Antagonisten, z.B. Caroverin, und Calmodulin-Inhibitoren die intrazelluläre Konzentration von Zytostatika (z.B. Vincristin und Adriamycin) erhöhen können, indem der Abfluss der Wirksubstanzen aus den Zellen verhindert wird. Solche Calcium-Antagonisten und Calmodulin-Inhibitoren wirken dabei als sogenannte Modulatoren. Gemäß ihren Untersuchungen sind die Calcium-Antagonisten nicht direkt zytotoxisch für die Tumorzellen. Dies wird auch von anderer Seite bestätigt (Menke et al., Dtsch. Med. Wschr. 113, 1728-1732, 1988).

Chan et al. (Gen. Pharmac. 25, 767-772, 1994) stellten fest, dass Oxysterole, d.h. Sterole, die ein oder mehrere Sauerstoffatome zusätzlich zu der 3β-Hydroxylgruppe aufweisen und vorwiegend aus Cholesterin synthetisiert werden, antiproliferative und teratogene Eigenschaften zeigen. Es wurde beschrieben, dass diese Wirkungen insbesondere die Bishemsuccinate von 7α-Hydroxycholesterol und 7β-Hydroxycholesterol zeigen, deren Wirkung darauf beruht, dass sie den Einbau von [³H]Thymidin in menschliche Rektumkrebszellen hemmen.

DE 27 08 666 A1 betrifft Arzneimittel mit immunoregulativer und antiphlogistischer Wirkung, wobei es sich bei den erfindungsgemäß verwendeten Verbindungen um 7-Hydroxycholesterin und 7-Ketocholesterin handelt. Die Verbindungen zeigen eine signifikante pharmakodynamische Wirkung als Immunregulativa mit immunsuppressiven Eigenschaften.

GB 2 053 924 A offenbart Hemiester von 7-oxygeniertem Cholesterin und deren Verwendung als Wirkstoff in pharmazeutischen Zusammensetzungen, wobei die Hemiester eine immunsuppressive Wirkung zeigen.

### Aufgabe der Erfindung

Obwohl, wie vorstehend beschrieben, schon eine Anzahl von Substanzen bekannt sind, denen eine positive Wirkung auf die Erhöhung der Immunleistungsfähigkeit zugeschrieben wird, gibt es nach wie vor einen Bedarf nach einer Zusammensetzung, die selbst bei schweren Erkrankungen (z.B. Krebs, Autoimmunerkrankungen, bakteriellen oder viralen Erkrankungen) oder bei erhöhter Stress- oder Umweltbelastung eine herkömmliche bzw. chemotherapeutische Therapie, die meist mit schweren Nebenwirkungen verbunden ist, reduziert oder sogar überflüssig macht.

Im Hinblick auf Krebs ist insbesondere ein Ziel, eine Wirkstoffzusammensetzung zur Erhöhung der Immunleistungsfähigkeit zur Verfügung zu haben. Es besteht auch der dringende Bedarf nach einer Therapiemöglichkeit falls es bereits zum Entstehen einer Zytostatika-Resistenz gekommen ist, die Tumoren weiter durch die körpereigene Immunabwehr zu bekämpfen.

Ferner sollen Nebenwirkungen von Strahlen- und Chemotherapie gemindert werden, bis hin zum strahleninduzierten Zweitkarzinom, Zelltransformationen und Metastasen. Darüber hinaus soll durch die Verwendung der Wirkstoffzusammensetzung ein Rückgang der Tumormarker und eine Verbesserung des Allgemeinbefindens hervorgerufen werden. Da viele Tumor-Patienten ausdrücklich auf eine traditionelle Behandlung durch Strahlen- oder Chemotherapie verzichten, ist es ein weiteres Ziel der vorliegenden Erfindung, die Wirkstoffzusammensetzung zur Monotherapie zu verwenden. Darüber hinaus soll durch die Verwendung der Wirkstoffzusammensetzung der Status nach Operation, Chemotherapie und/oder Bestrahlung verbessert werden.

Im Hinblick auf virale Erkrankungen ist ein Ziel der vorliegenden Erfindung, durch die Verwendung der Wirkstoffzusammensetzung die Virenlast zu senken, die Immunleistungsfähigkeit zu erhöhen und das Allgemeinbefinden zu verbessern. Darüber hinaus soll die Zahl der zellulären Bestandteile des Immunsystems erhöht werden. Insbesondere ist ein Ziel, die Virenlast bei chronischen Viruserkrankungen ohne die Gabe von hochaktiver antiretroviraler Therapie (*highly reactive anti-retroviral therapy*, HAART) und Interferon zu senken. Bei chronischen Viruserkrankungen sind noch Ziele, die Zeit bis zur Substitution mit HAART deutlich zu verlängern und die Verträglichkeit des HAART-Programms zu verbessern.

Die vorliegende Erfindung ist in den Ansprüchen definiert. Ausführungsbeispiele und gegenstände, die über den Inhalt der Ansprüche hinausgehen sind nicht als Teil der vorliegenden Erfindung anzuschen. Dies gilt insbesondere für die Verwendung zur Prävention und Behandlung Krebs.

Im Hinblick auf bakterielle Erkrankungen ist ein Ziel der vorliegenden Erfindung, durch die Verwendung der Wirkstoffzusammensetzung die Bakterienlast zu senken, die Immunleistungsfähigkeit zu erhöhen, insbesondere während der Verabreichung von Antibiotika, und das Allgemeinbefinden zu verbessern.

Im Hinblick auf Autoimmunerkrankungen ist ein Ziel der vorliegenden Erfindung, durch die Verwendung der Wirkstoffzusammensetzung die chronische Entzündung zu senken.

Im Hinblick auf erhöhte Stressbelastung ist ein Ziel der vorliegenden Erfindung, die körpereigene Immunaktivität, die Anzahl der NK-Zellen und Leukozyten zu erhöhen, sowie den Abbau der Peyerschen Plaques im Darm bei einem signifikanten Anstieg der körpereigenen Tumoraktivität und der Tumörnekrosefaktor-alpha- (TNF-alpha) Konzentration zu vermindern.

Im Hinblick auf erhöhte Umweltbelastung ist ein Ziel der vorliegenden Erfindung, die Immunleistungsfähigkeit zu erhöhen.

### Ausführliche Beschreibung der Erfindung

Erfindungsgemäß werden die obigen Ziele der Steigerung der körpereigenen Immunabwehrbei verschiedenen Erkrankungen einschließlich Stress- und Umweltbelastung durch eine pharmazeutische Zusammensetzung enthaltend als aktive Substanzen
(a) 1-(2-Diethylaminoethyl)-3-(*p*-methoxy-benzyl)-1,2-dihydrochinoxalin-2-on oder ein Derivat oder pharmazeutisch verträgliches Salz davon, und
(b) ein 7β-Hydroxycholesterol_oder ein pharmazeutisch verträgliches Salz davon das aus der Gruppe ausgewählt ist, die aus 7β-Hydroxycholesterol-bis-hemsuccinat-di-natriumsalz und 7β-Hydroxycholesterol-bis-hemsuccinat-diethanolaminoat besteht.
gelöst.

1-(2-Diethylaminoethyl)-3-(*p*-methoxy-benzyl)-1,2-dihydrochinoxalin-2-on (im Nachfolgenden "Caroverin" genannt) ist ein krampflösender Wirkstoff aus der Gruppe der Spasmolytika, ein Calcium- und Glutamatantagonist und Radikalfänger mit einer starken antioxidativen Wirkung auf Krankheitsformen, welche durch freie Radikalbildung im Rahmen des Sauerstoffmetabolismus verursacht werden. Ferner wirkt Caroverin neuroregenerativ und stimmungsaufhellend.

Caroverin weist einen ORAC-Wert *(Oxygen Radical Absorbing Capacity,* Redoxkapazität der Sauerstoffradikale) auf, der bis zu 1000fach höher gegenüber allen anderen bekannten Antioxidantien wie Vitamin C, E oder Glutathion ist.

Ein Vorteil von Caroverin ist, dass es seit Jahrzehnten als Spasmolytikum arzneimittelerechtlich registriert ist und auf Grund seiner ausgezeichneten Verträglichkeit geschätzt wird.

Die Wirkstoffkombination der vorliegenden Erfindung umfasst desweiteren ein 7β-Hydroxycholesterol-oder ein pharmazeutisch verträgliches Salz davon, das aus der Gruppe ausgewählt ist, die aus 7β-Hydroxycholesterol-bis-hemsuccinat-di-natriumsalz und 7β-Hydroxycholesterol-bis-hemsuccinat-diethanolaminoat besteht. Dabei können eine oder beide Hydroxylgruppen mit unterschiedlichen Substituenten, insbesondere mit Mono-, Bi- oder Tricarbonsäuren, substituiert sein. Durch geeignete Substituierung des 7β-Hydroxycholesterols kann dessen Wasserlöslichkeit, Resorptionsvermögen etc. beeinflusst werden. Entsprechend kann das eingesetzte Oxysterol eine Verbindung nachfolgender Strukturformeln sein:

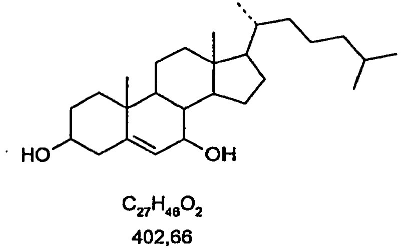

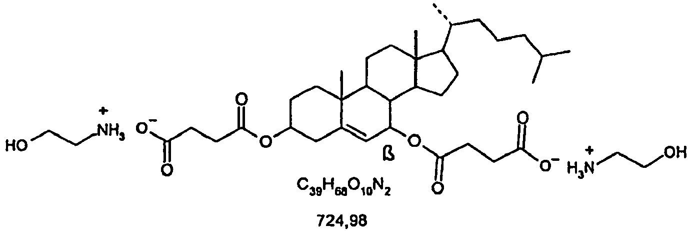

7β-Hydroxycholesterol ist in seiner natürlichen Form eine körpereigene Substanz der Thymusdrüse. Mit zunehmender Alterung verringert sich die Aktivität der Thymusdrüse. Insbesondere in Fällen von erhöhtem oxidativen Stress durch Krankheit oder durch Bestrahlung und Chemotherapie und der damit verbundenen starken Verminderung von Natürlichen Killerzellen (NK-Zellen) durch Knochenmarkschädigungen können die körpereigenen Abwehrmechanismen zur Funktion des zellulären Abwehrsystems nicht mehr greifen. Als körpereigene Substanz der Immunantwort und Aktivsubstanz der NK-Zellen wird 7β-Hydroxycholesterol von den Peptiden der NK-Zell-Oberfläche resorbiert.

Krebszellen benötigen zu ihrer ständigen Erneuerung und Vermehrung große Mengen an Cholesterol (Cholesterin), um ihre Zellmembran aufzubauen. Dieses Cholesterol wird von dem entsprechenden Lipoprotein niedriger Dichte (*Low Density Lipoproteins,* LDL) herbeigeschafft. Da 7β-Hydroxycholesterol eine größere Affinität zu LDL hat als Cholesterol, werden bei einer zusätzlichen Gabe von 7β-Hydroxycholesterol den Krebszellen vermehrt "falsche" Bausteine zugeführt. Dies führt in der Krebszelle rasch zu einem letalen Defizit an essentiellem Cholesterol. Die Folge ist eine kolloid-osmotisch induzierte Ruptur der Krebszelle. Zunächst blähen sich die Mitochondrien auf und es entstehen Vakuolen im Inneren der Krebszelle. Der Zellkern verkleinert sich und wird nekrotisiert. Es kommt zur natürlichen Apoptose der Krebszelle.

Wie bereits erwähnt, zeigte die Wirkstoffkombination aus einem 1-Diethylaminoethyl-3-Chinoxalin-2-on Derivat und einem Oxysterol erstmals überraschenderweise einen synergistischen Effekt bei der Behandlung von Krebs (siehe WO 2005/105089). Durch die Kombination wurde ein gut verträgliches Kombinationspräparat geschaffen. Das Kombinationspräparat wird gemäß der vorliegenden Erfindung nun auch zur Erhöhung der Immunleistungsfähigkeit bei Krebs, bakteriellen und viralen Erkrankungen, Autoimmunerkrankungen oder erhöhtem Stress verwendet.

Die Wirkstoffkombination aus Caroverin und 7β-Hydroxycholesterol wird im Folgenden "Synoverin" genannt. Synoverin enthält 20-200 mg Caroverin und 5-200 mg 7β-Hydroxycholesterol. Bevorzugt werden aber 20-120 mg Caroverin und 10-100 mg 7β-Hydroxycholesterol. Ganz besonders aber 20-60 mg Caroverin und 10-40 mg 7β-Hydroxycholesterol. Die gleichen Konzentrationen gelten für Ampullen, jedoch muss das Molekulargewicht der Salze entsprechend berücksichtigt werden.

Synoverin kann auf verschiedenen Wegen verabreicht werden, z.B. oral, parenteral, kutan, subkutan, intravenös, intramuskulär (i.m.) oder rektal. Bevorzugt ist die subkutane, orale oder rektale Verabreichung. Die Verbindung wird dem Patienten, der eine Therapie einer unter das Indikationsspektrum der erfindungsgemäßen Verbindungen fallenden Krankheit bedarf, über einen vom Arzt zu bestimmenden Zeitraum verabreicht. Die Verbindung kann sowohl Menschen als auch anderen Säugern verabreicht werden.

Die Dosierung der erfindungsgemäßen Verbindungen wird vom Arzt oder Heilpraktiker anhand der patientenspezifischen Parameter wie z. B. Alter, Gewicht, Geschlecht, Schwere der Erkrankung, etc. bestimmt..

Entsprechend der Art der Verabreichung wird das Medikament in geeigneter Weise formuliert, z.B. in Form von Lösungen bzw. Suspensionen, einfachen oder dragierten Tabletten, Hart- oder Weichgelatinekapseln, Suppositorien, Ovula, Injektionspräparaten, die nach üblichen galenischen Verfahren hergestellt werden.

Die erfindungsgemäßen Verbindungen können gegebenenfalls zusammen mit weiteren Wirkstoffen und mit in pharmazeutischen Zusammensetzungen üblichen Exzipientien formuliert werden. z.B. je nach herzustellendem Präparat Talk, Gummi arabicum, Lactose, Stärke, Magnesiumstearat, Kakaobutter, wässrige und nichtwässrige Träger, Fettkörper mit tierischem oder pflanzlichem Ursprung, Paraffinderivate, Glykole (insbesondere Polyethylenglycol), verschiedene Weichmacher, Dispergiermittel oder Emulgatoren, pharmazeutisch verträgliche Gase (z.B. Luft, Sauerstoff, Kohlendioxid usw.), Konservierungsstoffe.

Zur Herstellung flüssiger Präparate können Additive wie Natriumchloridlösung, Ethanol, Sorbit, Glycerin, Olivenöl, Mandelöl, Propylenglycol oder Ethylenglycol verwendet werden.

Bei der Verwendung von Infusions- oder Injektionslösungen sind diese sind bevorzugt wässrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Geringste Mengen an Antibiotika können auch zugesetzt werden, um die Beibehaltung der Sterilität zu gewährleisten.

Synoverin kann gemäß der vorliegenden Erfindung in einer Ampulle enthalten sein, wobei z.B. eine 2 ml Ampulle 5-20 mg 7β-Hydroxycholesterol, vorzugsweise als Salz, und 20-40 mg Caroverin, vorzugsweise als Hydrochlorid, enthält. Das Lösungsmittel ist vorzugweise gereinigtes Wasser. Darüber hinaus können die Bestandteile dieser Wirkstoffkombination in zwei Ampullen ("Ampulle 1" und "Ampulle 2") enthalten sein. Bevorzugt enthält Ampulle 1 15% Ethanol, 80% 1,2-Propylenglycol und 5% 7-β-Hydroxycholesterol. Ampulle 2 enthält vorzugweise 40 mg Caroverin auf 2 ml Wasser für Injektionszwecke.

Synoverin kann gemäß der vorliegenden Erfindung in einer Hartkapsel bzw. Kapsel enthalten sein. Die Kapsel kann Ascorbinsäure, 7-β-Hydroxycholesterol-bishemisuccinat-diethanolamin und Caroverin umfassen, vorzugsweise 200 mg Ascorbinsäure, 10 mg 7-β-Hydroxycholesterol-bishemisuccinat-diethanolamin und 60 mg Caroverin, vorzugsweise zur oralen Einnahme.

Synoverin kann gemäß der vorliegenden Erfindung in einer Salbe enthalten sein, vorzugsweise zur äußerlichen oder innerlichen Anwendung. Die Salbe umfasst vorzugsweise Silicea, Calendula Salbe, 7β-Hydroxycholesterol, Caroverin, Procainhydrochlorid und Nicotinsäureamid, vorzugsweise 70% Silicea, 20% Calendula Salbe, 5% 7β-Hydroxycholesterol, 5% Caroverin, 2% Procainhydrochlorid und 3% Nicotinsäureamid. Die Salbe kann darüber hinaus Zinkoxid umfassen.

Synoverin kann gemäß der vorliegenden Erfindung in einem Suppositorium enthalten sein. Das Suppositorium kann beispielsweise 7β-Hydroxycholesterol-bishemisuccinat-diethanolamin, Caroverin, Kakaobutter und Bienenwachs enthalten, vorzugsweise 10 mg 7 β-Hydroxycholesterol-bishemisuccinat-diethanolamin, 60 mg Caroverin, 1,6 g Kakaobutter und 0,3 Bienenwachs.

Erfindungsgemäß wird Synoverin 1-7 Mal pro Woche, vorzugsweise 3 Mal pro Woche, eine oder zwei Ampullen per Injektion, und/oder 1-7 Mal pro Woche, vorzugsweise täglich, vorzugsweise abends, ein Suppositorium und/oder 1-14 Mal pro Woche, vorzugsweise 2 Mal täglich, vorzugsweise morgens und abends, eine Hartkapsel appliziert. Besonders bevorzugt wird dreimal wöchentlich eine Ampulle und jeden Abend ein Suppositorium, oder zweimal täglich eine Hartkapsel, jeweils morgens und abends, verabreicht.

Synoverin als Synergie-Wirkstoff stellt ein Redoxsystem dar, bei dem sich 7β-Hydroxycholesterol als Oxidans bzw. aggressives freies Sauerstoffradikal (ROS, *Reactive Oxygene Species*) aus dem körpereigenen Fettstoffwechsel und Caroverin als Antioxidans synergistisch ergänzen, d.h. 7β-Hydroxycholesterol wirkt oxidierend und Caroverin reduzierend. Somit kann Synoverin insbesondere dann verwendet werden, wenn freie Radikale als Zwischenprodukte des Zellstoffwechsels entstehen, wie beispielsweise bei Stresssituationen, durch Krankheit und erhöhter Immunaktivität oder durch äußere Einflüsse wie Umweltbelastungen oder Chemo- und Strahlentherapie.

Bei Krebs handelt es sich gemäß der vorliegenden Erfindung um das unkontrollierte Wachstum und die Mutation (Wucherung) von neuem Gewebe, dass durch die Entartung körpereigener Zellen verursacht wird. Dieses bei Krebs entartete Gewebe wird gemäß der vorliegenden Erfindung bösartiger oder maligner Tumor genannt. Der Begriff Tumor bezeichnet allgemein eine Volumenzunahme von Gewebe, also eine Schwellung mit eingehender Platzforderung, die viele Ursachen haben kann. Der Ausdruck "Krebs" umfasst gemäß der vorliegenden Erfindung Sarkome, Karzinome, sowie Adenome, Lymphome und Leukämien, insbesondere Prostata-, Ovar-, Peritoneal- und Mammakarzinome und kanzerogene Hautveränderungen. Bei der Behandlung von Krebs wird Synoverin zum einen zur Prävention, Begleit- und Folgeanwendung eingesetzt und des Weiteren zur Durchbrechung von Resistenzen. Somit entwickeln die vorbestrahlten Tumorzellen keine Strahlenresistenz, wobei vorbestrahlte und nicht-vorbestrahlte Tumorzelllinien auf Synoverin in qualitativ und quantitativ gleicher Weise mit einer Proliferationshemmung reagieren.

Der Gleason-Wert (*Gleason-Score*) dient der histologischen, d.h. feingeweblichen Beurteilung von Krebs, insbesondere von Prostatakrebs (Humphrey P.A., Mod. Pathol. 17, 292-306, 2004). Dabei wird der sogenannte Entdifferenzierungsgrad (d.h. die Abweichung von normalem Gewebe) der häufigsten und der zweithäufigsten Zellpopulation des Tumors bewertet. Es werden jeweils Werte von 1 bis 5 vergeben. Je höher der Wert, desto höher ist der Grad der Entdifferenzierung. Die Angabe erfolgt immer nach dem Muster: Gleason-Wert 1 + -Wert 2 = Summe beide Werte.

Ferner erhöht Synoverin die Zellaktivität von Lymphozyten und Granulozyten. Dies bewirkt eine Steigerung der körpereigenen Immunabwehr, d.h. eine Erhöhung der Immunleistungsfähigkeit bei gleichzeitiger Senkung der Krebszellenaktivität. Es kommt infolge zu einer erhöhten Antikörperbildung, so dass neue gebildete Krebszellen schneller vom Immunsystem erkannt werden. Somit wird Synoverin gemäß der vorliegenden Erfindung auch zur Erhöhung der Immunleistungsfähigkeit bei Krebs verwendet.

Bei der Behandlung von Krebs bewirkt Synoverin als Oxidans durch die intrazelluläre Freisetzung von ROS ferner den natürlichen Zelltod von Krebszellen, die aufgrund der Instabilität ihrer Zellstruktur und ihres Zellstoffwechsels den ROS-Attacken nicht standhalten können. Gleichzeitig schützt es als starkes Antioxidans und Reduktionsmittel in bis zu 1000facher Wirkung gegenüber bisher bekannter Systemen gesunde Zellen vor den ROS-Attacken und gefürchteten Nebenwirkungen bei der Strahlen- und Chemotherapie. Beispielsweise verhindert Synoverin ein strahleninduziertes Zweitkarzinom, Zelltransformationen, Rezidiven und Metastasen. Somit wird Synoverin auch zur Minderung von Nebenwirkungen von Strahlen- und Chemotherapie verwendet.

Außerdem bewirkt Synoverin :
- einen Rückgang der Tumormarker, beispielsweise PSA (Prostataspezifisches Antigen), insbesondere FPSA (Freies PSA) oder CPSA (Complexiertes PSA), CEA (Carcinoembryonales Antigen), CA bzw. Ca (Cancer Antigen), insbesondere Ca 15-3 und Ca 12-5;
- einen Rückgang der Entzündungsmarker, beispielsweise BSG (Blutkörper-Senkungsgeschwindigkeit), CRP (C-reaktives Protein), CPK (Creatinphosphokinase),
- eine positive Beeinflussung der Anzahl von spezifischen Antikörpern, insbesondere lgM- und lgA-Antikörper, EBV-VCA-lgM bzw. EBV-VCA-lgG (lgG- bzw. lgM-Antikörper gegen das virale Capsid-Antigen des Epstein-Barr Virus), EBNA-lgG (lgG-Antikörper gegen das nukleäre Antigen des Epstein-Barr Virus), Herpes Zoster lgG (lgG-Antikörper gegen das Virus Herpes Zoster), Borrelien lgG-Antikörper;
- eine Verbesserung des Blutbildes, Leber- und/oder Nierenprofils, beispielsweise zum Rückgang des Hydroxylamins, der Harnsäure, der alkalischen Phosphatase (AP), des Bilirubins, der Gamma-Glutamyltranspeptidase (GGT) und/oder
- eine Verminderung der Virenlast,
- eine Verminderung der Bakterienanzahl.

Der Rückgang des Hydroxylamins wird über den Neunhoeffer-Test detektiert. Der Neunhoeffer-Test dient zur Erkennung von Stoffwechselstörungen. Es handelt sich um eine Bestimmung aus dem Harn. Bei der Harnuntersuchung werden Stoffwechselrückstände gemessen, die bei einem Gesunden nicht vorhanden sind (Neunhoeffer O., SANUM-Post 50/2000, 6-7).

Darüber hinaus bessert sich durch die Verwendung von Synoverin das Allgemeinbefinden, insbesondere während der Behandlung von Krebs.

Viele Tumorpatienten verzichten ausdrücklich auf eine traditionelle Behandlung durch Strahlen- und Chemotherapie. In diesem Fall ist eine vom Patienten ausdrücklich gewünschte, alternative Monotherapie gefragt. Synoverin kann auch zur Monotherapie verwendet werden.

Außerdem wird durch die Verwendung von Synoverin nach Operation, Chemotherapie und/oder Bestrahlung der Status verbessert.

Als Oxidans bewirkt es durch die intrazelluläre Freisetzung von ROS auch den natürlichen Zelltod von Viren, insbesondere menschliches Immunschwächevirus (*human immunodeficiency* virus, HIV- 1 oder -2), Hepatitis-A-, Hepatitis-B- oder Hepatitis-C-Virus, Herpes-Virus (z.B. Herpes zoster), Epstein-Barr-Virus (EBV), Varizella-Zoster-Virus, oder Cytomegalievirus (CMV), die aufgrund der Instabilität ihrer Zellstruktur und ihres Zellstoffwechsels den ROS-Attacken nicht standhalten können. Gleichzeitig schützt es als starkes Antioxidans und Reduktionsmittel in bis zu 1000facher Wirkung verglichen mit bisher bekannten Systeme gesunde Zellen vor den ROS-Attacken und seinen gefürchteten Nebenwirkungen. Somit wird Synoverin gemäß der vorliegenden Erfindung zur Senkung der Virenlast, Erhöhung der Gesamtleukozytenzahl und/oder der T4-Zellen verwendet. Insbesondere wird durch die Verwendung von Synoverin bei chronischen Viruserkrankungen, insbesondere bei Erkrankungen mit HIV, Hepatitis, EBV, Herpes Zoster oder Cytomegalie, die Virenlast ohne die Gabe von HAART und/oder Interferon gesenkt.

Ferner erhöht Synoverin die Zellaktivität von Lymphozyten und Granulozyten. Dies bewirkt eine Steigerung der körpereigenen Immunabwehr, d.h. eine Erhöhung der Immunleistungsfähigkeit bei gleichzeitiger Senkung der Virenaktivität bzw. der chronischen Entzündung. Es kommt infolge zu einer erhöhten Antikörperbildung, so dass neue Viren schneller vom Immunsystem erkannt werden. Somit wird Synoverin gemäß der vorliegenden Erfindung auch zur Erhöhung der Immunleistungsfähigkeit bei Viruserkrankungen verwendet.

Darüber hinaus bessert sich durch die Verwendung von Synoverin das Allgemeinbefinden, auch während der Behandlung von Viruserkrankungen.

Außerdem verlängert die Verwendung von Synoverin bei chronischen Viruserkrankungen, insbesondere mit HIV, die Zeit bis zur Substitution mit HAART deutlich bzw. verbessert die Verträglichkeit des HAART-Programms deutlich. Somit wird Synoverin gemäß der vorliegenden Erfindung bei chronischen Viruserkrankungen zur Verlängerung der Zeitspanne bis zur HAART-Substitution und zur Verbesserung der Verträglichkeit des HAART-Programms verwendet. Eine Kombination von Synoverin mit den Arzneimitteln des HAART-Programms ist erfindungsgemäß angezeigt.

Als Oxidans bewirkt Synoverin durch die intrazelluläre Freisetzung von ROS auch gegen Bakterien, die aufgrund der Instabilität ihrer Zellstruktur und ihres Zellstoffwechsels den ROS-Attacken nicht standhalten können. Gleichzeitig schützt es als starkes Antioxidans und Reduktionsmittel in bis zu 1000facher Wirkung bisher bekannter Systeme gesunde Zellen vor den ROS-Attacken und seinen gefürchteten Nebenwirkungen. Zu den pathogenen, insbesondere humanpathogenen Bakterien zählen gemäß der vorliegenden Erfindung Streptokokken und deren Toxine (z.B. Streptokokken der Serogruppe D oder Beta-hämolysierende Streptokokken der Serogruppe B, insbesondere *Streptococcus. mutans, S. salivarius, S. pneumoniae, S*. *pyogenes,* S. *viridans*), Staphylokokken und deren Toxine (z.B. *Staphylococus aureus, S. epidermidis* und *S*. *saprophyticus, S. intermedius),* Enterokokken, Klebsiellen, *Haemophilus influenzae,* Rickettsien, Legionellen, Mykobakterien, Mykoplasmen, Ureaplasmen, Pseudomonaden, Bordetellen (z.B. *Bordetella pertussis*), Corynebakterien (z.B. *Corynebacterium diphtheriae, C. minutissimum*), Chlamydien, Campylobakterien (z.B. *Campylobacter coli, C. jejuni*), *Escherichia coli,* Proteus, Salmonellen, Shigellen, Yersinien, Vibrionen, Clostridien, Listerien, Borrelien, *Treponema pallidum,* Brucellen, Francisellen oder Leptospira.

Ferner erhöht Synoverin die Zellaktivität von Lymphozyten und Granulozyten. Dies bewirkt eine Steigerung der körpereigenen Immunabwehr, d.h. eine Erhöhung der Immunleistungsfähigkeit bei gleichzeitiger Senkung der Bakterienaktivität. Es kommt infolge zu einer erhöhten Antikörperbildung, so dass neue Bakterien schneller vom Immunsystem erkannt werden. Somit wird Synoverin gemäß der vorliegenden Erfindung auch zur Erhöhung der Immunleistungsfähigkeit bei bakteriellen Erkrankungen verwendet.

Darüber hinaus bessert sich durch die Verwendung von Synoverin das Allgemeinbefinden, auch während der Behandlung von bakteriellen Erkrankungen.

Darüber hinaus erhöht Synoverin die Immunleistungsfähigkeit und senkt die TNF-alpha Aktivität, so dass eine überschießende Immunreaktion des Körpers verhindert wird. Dies wirkt sich bei Autoimmunerkrankungen positiv aus, insbesondere bei Hashimoto-Thyreoiditis, Morbus Basedow, Morbus Crohn, Rheuma, Psoriasis, Fibromyalgie und Multiple Sklerose.

Außerdem wird Synoverin gemäß der vorliegenden Erfindung als Präventionstherapie bei Absinken der Immunleistungsfähigkeit in Folge erhöhter Stressbelastung durch berufliche Überforderung oder Hochleistungssport eingesetzt. Hier sorgt Synoverin zu einer Verminderung des Absinkens der körpereigenen Immunaktivität, zu einer Erhöhung der Anzahl von NK-Zellen und Leukozytenzahl, zu einer Verminderung des Abbaus der Peyerschen Plaques im Darm und des signifikanten Anstiegs der körpereigenen Tumoraktivität und der TNF-alpha Konzentration. Deren Folge wären chronische und reaktivierte Erkrankungen.

Ferner beeinflusst Synoverin gemäß der vorliegenden Erfindung das Absinken der Immunleistungsfähigkeit bei erhöhter Umweltbelastung positiv. Durch Umweltbelastungen durch beispielsweise organische Stoffe, z.B. Lindane, Pentachlorphenol (PCP), Arzneimittel im Trinkwasser, oder Schwermetalle, z.B. aus Feinstaub, Böden, Wasser, Industrie, Zahn-Amalgam, gelangen freie Radikale von außen in den Körper, die zu einem Absinken der Immunleistungsfähigkeit führen.

Die Begleittherapie zur Verabreichung von Synoverin umfasst vorzugsweise einen gesunden Lebensstil, die Substitution mit Mikronährstoffen und/oder eine psychologische Begleitung.

Der Ausdruck "gesunder Lebensstil" umfasst zum einen vollwertige, Basen-überschüssige Ernährung, täglich frisches Obst, Gemüse und Salate, täglich stilles Wasser oder Kräutertees (mindestens 3 ml pro Kilogramm Körpergewicht), täglich mindestens 7 Stunden Schlaf und/oder dreimal wöchentlich 30 bis 40 Minuten Ausdauersport, z.B. Walking, Radfahren oder Schwimmen. Bevorzugt ist eine basische gluten- und/oder lactosefreie Ernährung.

Die "Substitution mit Mikronährstoffen" umfasst Phytotherapie, die Substitution mit Mineralstoffen (z.B. Spurenelemente), essentiellen Aminosäuren (z.B. Arginin, Prolin, Lysin, Cystein, Ornithin, 5-Hydroxytryptophan) und Fettsäuren zur Regulation der defizitären Abwehrlage, insbesondere Substitution mit Selen, Zink, Kupfer, Vitamin C, Vitamin E, Vitamin B12, B6, B2, B1 und Folsäure, einem Enzym(vitalstoff)präparat, einem Probiotikum zum Aufbau der Darmflora und/oder einem diätetischen Lebensmittel (z.B. Orthomol Immun^{®}, Almased^{®} oder Fresubin^{®}).

Als "Phytotherapie" wird die Verwendung von Heilpflanzen als Arzneimittel bezeichnet. Beispiele für Heilpflanzen sind Chinarinde (*Cortex chinae*), Eichenrinde (*Quercus* cortex), Faulbaumrinde (*Cortex frangulae*), Seifenrinde (Cortex *quillaiae*), Weidenrinde (*Salicis cortex*), Zimtrinde (*Cortex Cinnamomi*), Lindenblüte (*Flos tiliae*), Kamillenblüte (*Matricariae flos, Flos chamomillae*), Heublumen, Grasblüten (*Graminis flos*), Holunderblüte (*Flores sambuci*), Erdbeerblatt (*Folium fragariae*), Spitzwegerich (*Folium plantaginis*), Salbeiblatt (*Folium salviae*), Kümmel (*Fructus carvi*), Fenchel (*Fructus foeniculi*), Hagebutte (*Fructus cynosbati*), Wacholderbeere (*Fructus junipen*), Brennnessel (*Herba urticae*), Tausendgüldenkraut (*Herba centaun*), Wermut (*Herba absinthii*), Zinnkraut (Schachtelhalm) (*Herba equiseti*), Baldrianwurzel (*Radix valerianae*), Gelber Enzian (*Radix Gentianae*), Ginseng (*Ginseng radix*), Ingwer (*Zingiberis rhizoma*), Nieswurz (*Hellebori rhi*zoma), Veilchenwurzel, ungeschält (*Iridis cum cortice rhizoma*), Kürbiskern (*Cucurbitae semen*), Leinsamen (*Semen lim*) oder Muskatnuss (*Myristicae semen*).

Als Enzymvitalstoffpräparat ist beispielsweise Innovazym^{®} (Fa. InnovaVital GmbH, Grassau, Deutschland), enthaltend pro 7 g, entsprechend 7 Tabs, Enzyme (120 mg Bromelain, 300 mg Papain, 30 mg Lysozym); Vitalstoffe (200 mg Magnesium, 10 g Zink, 100 µg Selen); Bioflavonoide (500 mg sekundäre Pflanzenstoffe); Q 10 (30 mg Coenzym Q10); Vitamine (334 µg Provitamin A, 3 mg Vitamin B1, 3 mg Vitamin B2, 6 mg Vitamin B6, 9 µg Vitamin B12, 600 µg Folsäure, 600 mg Vitamin C, 60 mg Vitamin E, 1000 µg Vitamin A) bevorzugt.

Ferner ist Wobenzym^{®} P bzw. Wobenzym^{®} P TMR (Mucos Pharma GmbH & Co. KG, Berlin, Deutschland) als Enzympräparat bevorzugt. Wobenzym^{®} P bzw. Wobenzym^{®} P TMR beinhalten eine Kombination der Enzyme Bromelain, Papain und Rutosid.

Darüber hinaus ist auch Phlogenzym^{®} der Fa. Mucos Pharma GmbH & Co. KG, Berlin, Deutschland als Enzympräparat bevorzugt, enthaltend Bromelain, Trypsin und Rutosid als arzneilich wirksame Bestandteile. Als Probiotikum ist Bactoflor^{®} der Fa. Intercell Pharma GmbH, Höhenkirchen, Deutschland, enthaltend probiotische Bakterienkulturen in einer Konzentration von 20 x 10⁹ koloniebildenden Einheiten, Inulin, Calciumcarbonat, pflanzliche Cellulosekapsel, Kartoffelstärke, Ascorbinsäure und pflanzliches Magnesiumstearat besonders bevorzugt. Als Bakterienkulturen sind *Bifidobakterium bifidum, B. breve, B. longum*, *Lactobacillus acidophilus*, *L. casei, L. delbrueckü* subsp. *bulgaricus, L. fermentum, L. rhamnosus, L. plantarum* und *Lactococcus lactis* bevorzugt.

Als Probiotikum ist auch Mutaflor^{®} der Fa. Ardeypharm GmbH, Herdecke, Deutschland, enthaltend *Escherichia coli* als Wirkstoff, insbesondere *E*. *coli* Stamm Nissle 1917, besonders bevorzugt.

Darüber hinaus ist auch Paidoflor^{®} als Probiotikum der Fa. Ardeypharm GmbH, Herdecke, Deutschland, enthaltend *Lactobacillus acidophilus* (10⁹-10¹⁰ lebensfähige Bakterien pro g) als arzneilich wirksamen Bestandteil und Riboflavin, Nicotinsäure, Magnesiumstearat, Magnesiumsulfat 7 H₂O, Mangan(II)sulfat, Lactose und Lactose-Monhydrat, bevorzugt.

Als diätetisches Lebensmittel ist Orthomol Immun^{®} (Fa. Orthomol pharmazeutische Vertriebs GmbH, Langenfeld, Deutschland), enthaltend pro 100 g Vitamine (3 mg Vitamin A; 6,3 g Vitamin C; 1 g Vitamin E; 167 mg Vitamin B1; 167 mg Vitamin B2; 400 mg Nicotinamid; 133 mg Vitamin B6; 40 µg Vitamin B12; 400 µg Vitamin K1; 33 µg Vitamin D3; 5,3 mg Folsäure; 120 mg Panthothensäure; 1,1 mg Biotin), Spurenelemente (333 µg Selen; 53 mg Eisen; 67 mg Zink; 13 mg Mangan; 3,3 mg Kupfer; 200 µg Chrom; 400 µg Molybdän; 1 mg Jod) und sekundäre Pflanzenstoffe (33 mg Citrus-Bioflavonoide, 33 mg gemischte Carotinoide, enthaltend Beta-Carotin, Lutein und Lycopin) bevorzugt.

Darüber hinaus sind Almased^{®} (Fa. Almased Wellness GmbH, Bienenbüttel, Deutschland) und Fresrubin^{®} (Fa. Fresenius, Bad Homburg, Deutschland) als diätetische Lebensmittel bevorzugt.

Der Begriff "psychologische Betreuung" umfasst eine verbale und/oder medikamentöse Psychotherapie. Die Konfrontation mit der Diagnose einer schweren Krankheit, verbunden mit unausgesprochenen Ängsten und seelischen Konflikten tragen zur belastenden Situation von Patienten bei. Der Bedarf an professioneller Begleitung ist groß, auch wenn er oftmals nicht eingestanden wird. Eine Empfehlung des medizinischen Therapeuten, psychologische Betreuung in Anspruch zu nehmen, kann den Patienten ermutigen und den Verlauf seines Behandlungsprozesses in positiver Weise beeinflussen.

### Figurenbeschreibung

Figur 1 zeigt den Hydroxylaminwert in Abhängigkeit zur Zeit in Monaten während der Behandlung mit Synoverin.
Figur 2 zeigt den CEA- und Ca 15-3-Wert in Abhängigkeit zur Zeit in Monaten während der Behandlung mit Synoverin.
Figur 3 zeigt den CEA-, Ca 15-3-, Ca 12-5-, CRP- und Harnsäure-Wert in Abhängigkeit zur Zeit in Monaten während der Behandlung mit Synoverin, wobei der Ca 15-3-Wert mit 10 (x10) und der Ca 12-5-Wert mit 1000 (x1000) multipliziert werden muss.
Figur 4 zeigt den Hydroxylaminwert in Abhängigkeit zur Zeit in Monaten während der Behandlung mit Synoverin.
Figur 5 zeigt den PSA-, CPSA-, CRP- und Harnsäure-Wert in Abhängigkeit zur Zeit in Monaten während der Behandlung mit Synoverin.
Figur 6 zeigt den Hydroxylaminwert in Abhängigkeit zur Zeit in Monaten während der Behandlung mit Synoverin.
Figur 7 zeigt den PSA- und AP-Wert in Abhängigkeit zur Zeit in Monaten während der Behandlung mit Synoverin.

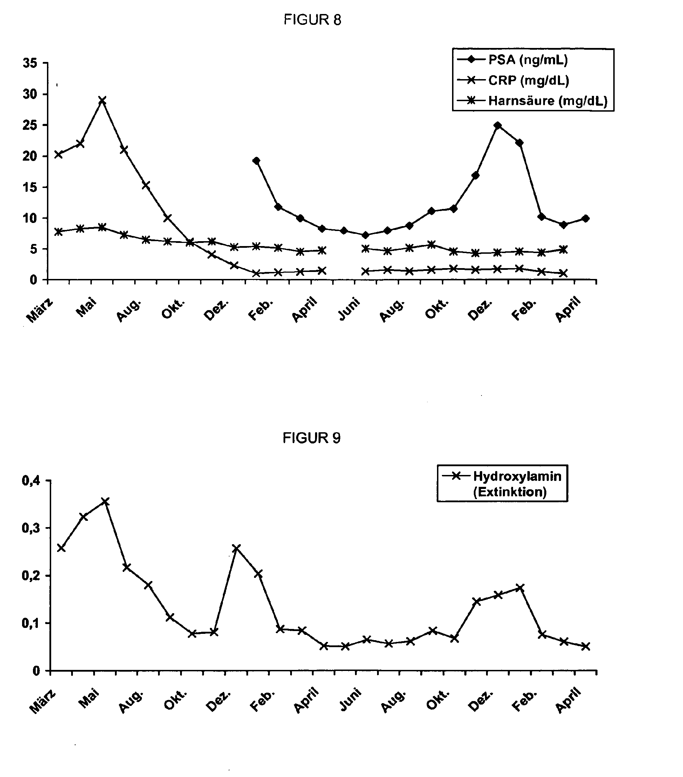

Figur 8 zeigt den PSA-, CRP- und Harnsäure-Wert in Abhängigkeit zur Zeit in Monaten während der Behandlung mit Synoverin.
Figur 9 zeigt den Hydroxylaminwert in Abhängigkeit zur Zeit in Monaten während der Behandlung mit Synoverin.
Figur 10 zeigt den EBV-VCA-IgM-, EBV-VCA-IgG- und EBNA-1-IgG-Wert in Abhängigkeit zur Zeit in Monaten während der Behandlung mit Synoverin.
Figur 11 zeigt den Bilirubin gesamt -, CRP-, Harnsäure- und GGT-Wert in Abhängigkeit zur Zeit in Monaten während der Behandlung mit Synoverin, wobei der GGT-Wert mit 10 (x10) multipliziert werden muss.
Figur 12 zeigt den CD4-Zellen-, CD8-Zellen-, CD3+/HLA DR+- und NK-Zellen-Wert in Abhängigkeit zur Zeit in Monaten während der Behandlung mit Synoverin.
Figur 13 zeigt den Hydroxylaminwert in Abhängigkeit zur Zeit in Monaten während der Behandlung mit Synoverin.

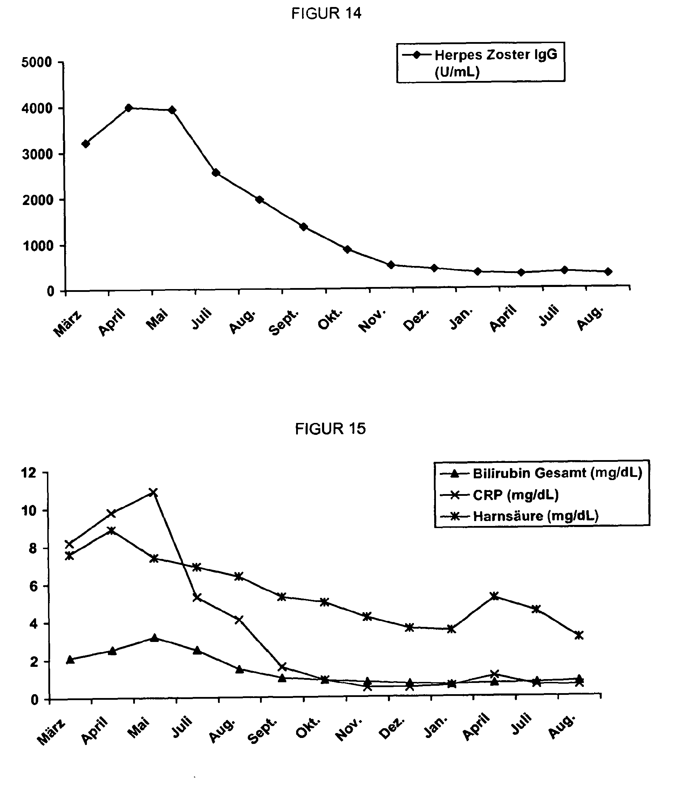

Figur 14 zeigt den Herpes Zoster IgG-Wert in Abhängigkeit zur Zeit in Monaten während der Behandlung mit Synoverin.
Figur 15 zeigt den Bilirubin gesamt -, CRP- und Harnsäure-Wert in Abhängigkeit zur Zeit in Monaten während der Behandlung mit Synoverin.

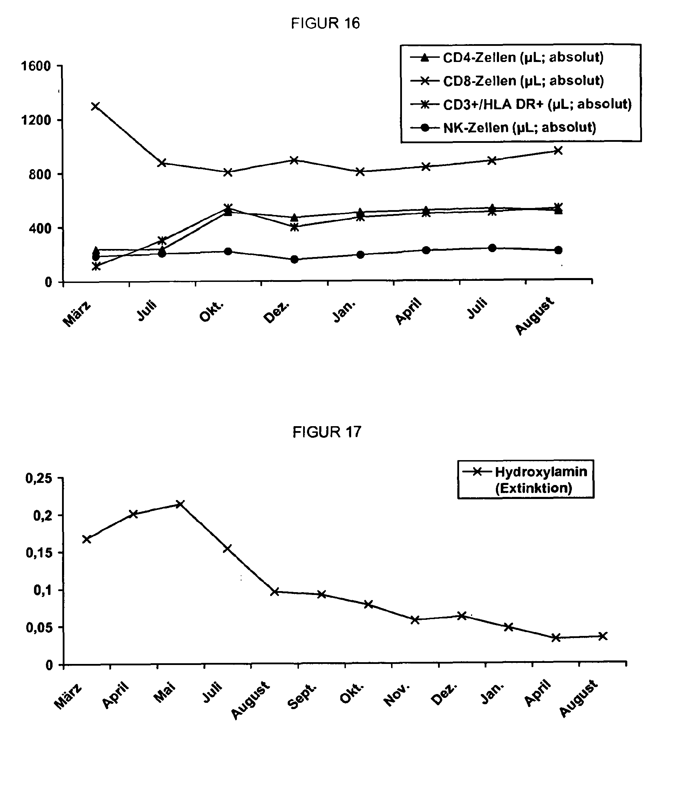

Figur 16 zeigt den CD4-Zellen-, CD8-Zellen, CD3+/HLA DR+- und NK-Zellen-Wert in Abhängigkeit zur Zeit in Monaten während der Behandlung mit Synoverin.
Figur 17 zeigt den Hydroxylaminwert in Abhängigkeit zur Zeit in Monaten während der Behandlung mit Synoverin.
Figur 18 zeigt die HIV-Viruslast in Abhängigkeit zur Zeit in Monaten während der Behandlung mit Synoverin.
Figur 19 zeigt den Bilirubin gesamt -, CRP- und Harnsäure-Wert in Abhängigkeit zur Zeit in Monaten während der Behandlung mit Synoverin.
Figur 20 zeigt den CD4-Zellen-, CD8-Zellen-, CD3+/HLA DR+- und NK-Zellen-Wert in Abhängigkeit zur Zeit in Monaten während der Behandlung mit Synoverin.
Figur 21 zeigt den Hydroxylaminwert in Abhängigkeit zur Zeit in Monaten während der Behandlung mit Synoverin.
Figur 22 zeigt die HIV-Viruslast in Abhängigkeit zur Zeit in Monaten während der Behandlung mit Synoverin.
Figur 23 zeigt den Bilirubin gesamt -, CRP- und Harnsäure-Wert in Abhängigkeit zur Zeit in Monaten während der Behandlung mit Synoverin.
Figur 24 zeigt den CD4-Zellen-, CD8-Zellen, CD3+/HLA DR+- und NK-Zellen-Wert in Abhängigkeit zur Zeit in Monaten während der Behandlung mit Synoverin.
Figur 25 zeigt den Hydroxylaminwert in Abhängigkeit zur Zeit in Monaten während der Behandlung mit Synoverin.
Figur 26 zeigt den CPK-, IgA- und IgM-Wert in Abhängigkeit zur Zeit in Monaten während der Behandlung mit Synoverin.
Figur 27 zeigt den Bilirubin gesamt -, CRP-, Harnsäure- und Leukozyten-Wert in Abhängigkeit zur Zeit in Monaten während der Behandlung mit Synoverin.
Figur 28 zeigt den CD4-Zellen-, CD8-Zellen-, CD3+/HLA DR+- und NK-Zellen-Wert in Abhängigkeit zur Zeit in Monaten während der Behandlung mit Synoverin.
Figur 29 zeigt den Hydroxylaminwert in Abhängigkeit zur Zeit in Monaten während der Behandlung mit Synoverin.

### Beispiele

### BEISPIEL 1

Bei einer Patientin, 45 Jahre alt, wurde ein Mammakarzinom, das sich im Stadium 3 nach Biraz befand und eine Tumorgröße von 2 cm hatte, diagnostiziert. Die Tumormarker waren unauffällig, allerdings ergab der Neunhoeffer-Test erhöhtes Hydroxylamin und ein Mineralstoffmangel wurde diagnostiziert; das sonstige Labor war unauffällig.

Mit der Behandlung ausschließlich mit Synoverin wurde sofort begonnen. Es wurde dreimal täglich 1 Kapsel Synoverin verabreicht, wobei begleitend eine Substitution mit Mineralstoffen, Vitaminen und Probiotika erfolgte.

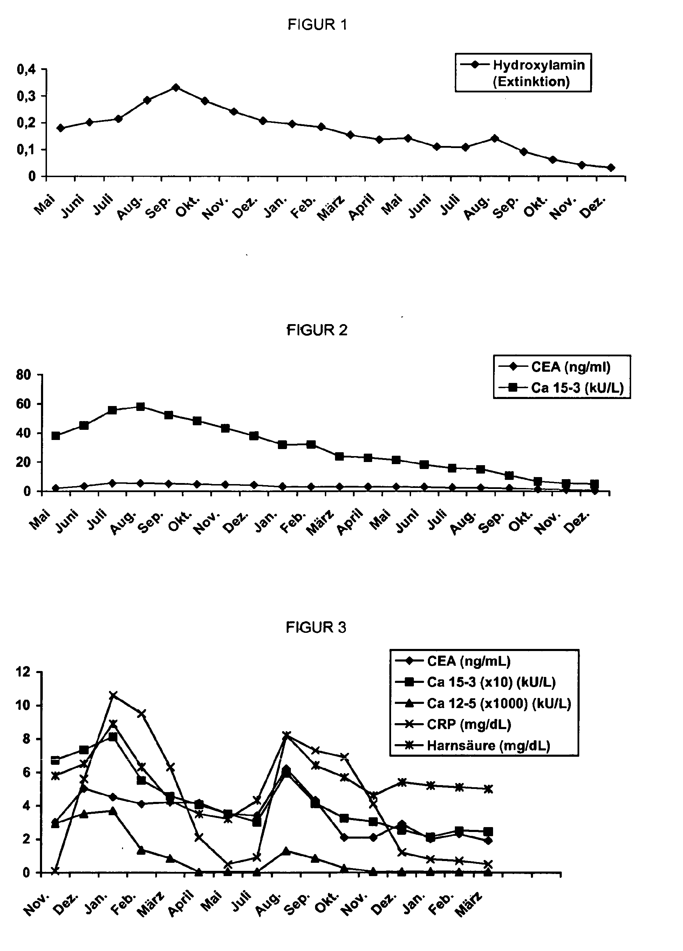

Vier Monate nach Behandlungsbeginn wurde ein kontinuierlicher Rückgang der Tumorgröße, der Tumormarker und des Hydroxylaminwertes beobachtet. Nach ungefähr eineinhalb Jahren ab Behandlungsbeginn war der Befund unauffällig (siehe Fig. 1 und 2), im MRT (Magnetresonanztomographie) kein Tumor mehr sichtbar und es gab keine Indikation zur OP (Operation) mehr. Die Patientin war während des Therapieverlaufs immer in sehr guter Allgemeinverfassung, wobei sich durch die Gabe von Synoverin das Allgemeinbefinden deutlich gebessert hat.

Die Behandlung mit Synoverin führte bei dieser Patientin dazu, dass das frühproliferative Geschehen signifikant zurückgegangen ist. Es gab einen deutlichen Korrelationseffekt zwischen Tumorgröße, Tumormarker, Hydroxylaminwert und/oder Allgemeinbefinden. Zur Prophylaxe war eine Fortführung der Synoverin-Therapie notwendig.

### BEISPIEL 2

Bei einer Patientin, 60 Jahre alt, mit einem primären Peritonealkarzinom (Adeno) ergab die Untersuchung nach CHE und einer Omenektomie vier Jahre vor dem Behandlungsbeginn mit Synoverin Lymphknotenrezidiven, supraclaviculär links. Bei der Untersuchung im Rahmen der vorliegenden Erfindung wurden pathologische Lymphknoten, mediastinal, prä- und paratracheal, sowie links axillär und rechts supraclaviculär und postradiogene pneumonitische Oberlappenveränderungen links festgestellt. Außerdem wurde eine leichte Arteriosklerose der Aorta und eine diffuse Karzinose mit Punktum Maximum unter dem Duodenum an der Mesenterialwurzel, subhepatisch, diagnostiziert. Ferner ergab die Untersuchung diffuse pathologische Lymphadenopathie, auch entlang der Beckengefäße, wobei auch die Darmlumina pathologisch erweitert waren. Darüber hinaus wurden knöcherne Strukturen ohne suspekte Arrosionen diagnostiziert. Allerdings ergab die Untersuchung keine Aszites und einen unauffälligen Befund der Nieren, Leber, Galle und Pankreas. Die Tumormarker Ca 12-5, Ca 15-3, CEA waren aber erhöht und der Neunhoeffer-Test zeigte stark erhöhtes Hydroxylamin. Außerdem wurde ein Mineralstoffmangel, erhöhte Harnsäurewerte, Neutrophile 70, und Lymphozyten 15 festgestellt, wobei der CRP-Wert normal und das sonstige Labor unauffällig war. Auf ausdrücklichen Wunsch des Patienten wurde keine weitere OP, Chemo- oder Strahlentherapie durchgeführt.

Mit der Behandlung ausschließlich mit Synoverin wurde sofort begonnen. Über 20 Wochen wurde zweimal täglich 1 Suppositorium Synoverin und zweimal täglich 1 Kapsel Synoverin verabreicht. Anschließend wurde abends 1 Suppositorium Synoverin und zweimal täglich 1 Kapsel Synoverin verabreicht. Begleitend erfolgte eine Substitution mit Mineralstoffen, Vitaminen, Enzymen und Probiotika und die Organfunktionen wurden zusätzlich durch Phytotherapie unterstützt. Außerdem erfolgte ein Ernährungsumstellung auf basische gluten-/lactosefreie Kost. Bei der ambulanten Behandlung wurde Synoverin über 20 Wochen täglich i.m. verabreicht, danach dreimal wöchentlich und begleitend erfolgte eine Substitution mit Vitaminen. Neun Monate nach Behandlungsbeginn wurde die Dosierung auf die der ersten 20 Wochen erhöht.

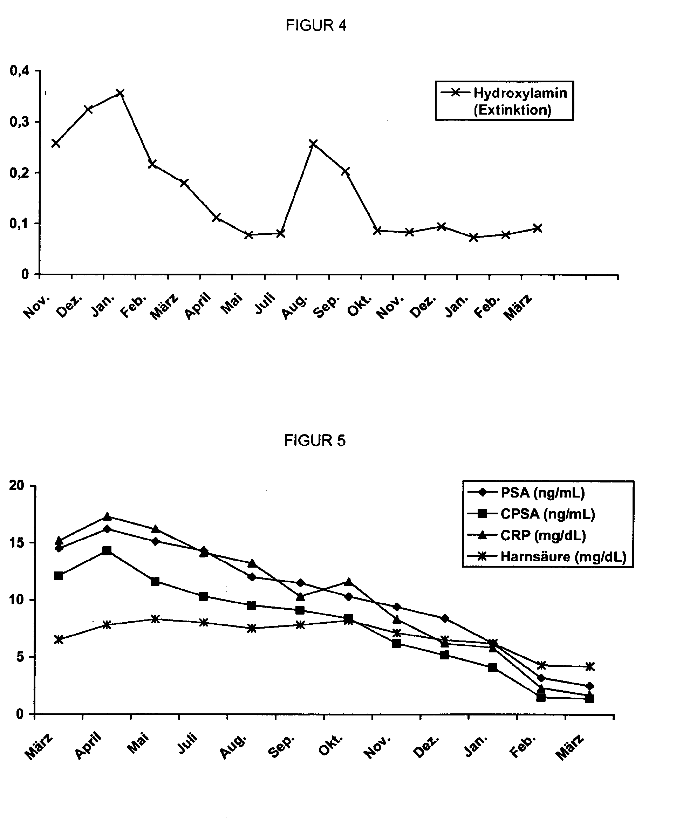

Zwei Monate nach Behandlungsbeginn gingen die erhöhten Hydroxylamin-, Harnsäure-, CRP- und Tumormarkerwerte signifikant zurück (siehe Fig. 3 und Fig. 4). Ein kontinuierlicher Rückgang der Tumorwerte und eine stetige Besserung im Befund zeigten sich 1 Jahr nach Behandlungsbeginn. Im CT (Computertomographie) reduzierte sich die Tumormasse sichtbar. Ungefähr eineinhalb Jahre nach Behandlungsbeginn wurden keine neuen Rezidive beobachtet und es gab keine weitere Indikation zur OP oder Chemotherapie mehr. Bezüglich des Allgemeinbefindens, war die allgemeine Verfassung der Patientin während der ersten zwei Monate schlecht, sie litt unter Müdigkeit, Hautjucken und Appetitlosigkeit. Danach war sie insgesamt in einem stabilen Zustand und auch der erneute Anstieg der Tumormarker führte zu keiner Verschlechterung des Allgemeinbefindens.

Die Behandlung mit Synoverin führte bei dieser Patientin dazu, dass das proliferative Geschehen signifikant zurückgegangen ist, mit einem Korrelationseffekt zwischen Tümormarker, Hydroxylaminwert und/oder Allgemeinbefinden. Zur Prophylaxe war eine Fortführung der Synoverin-Therapie notwendig.

### BEISPIEL 3

Bei einem Patienten, 51 Jahre alt, wurde ein Prostatakarzinom (Adeno), pT1c (Primärtumor mit einer Größe von 1 cm bis 2 cm in seiner größten Ausdehnung), mit einem Gleason-Score von 3+5=8 diagnostiziert. Die Untersuchung im Rahmen der vorliegenden Erfindung ergab, dass der Tumormarker PSA erhöht war und der Neunhoeffer-Test hatte erhöhtes Hydroxylamin als Ergebnis. Außerdem wurden ein Mineralstoffmangel und ein erhöhter Harnsäure-Wert festgestellt, wobei das sonstige Labor unauffällig war. Auf ausdrücklichen Wunsch des Patienten wurde keine OP, Chemö- oder Strahlentherapie durchgeführt.

Mit der Behandlung ausschließlich mit Synoverin wurde sofort begonnen. Es wurden zweimal täglich 1 Suppositorium Synoverin und begleitend Mineralstoffe, Aminosäuren, Vitamine, Enzyme und Probiotika verabreicht. Außerdem erfolgte eine Ernährungsumstellung auf basische gluten-/lactosefreie Kost. Bei der ambulanten Behandlung wurde Synoverin täglich i.m. verabreicht und begleitend erfolgte eine Substitution mit Vitaminen.

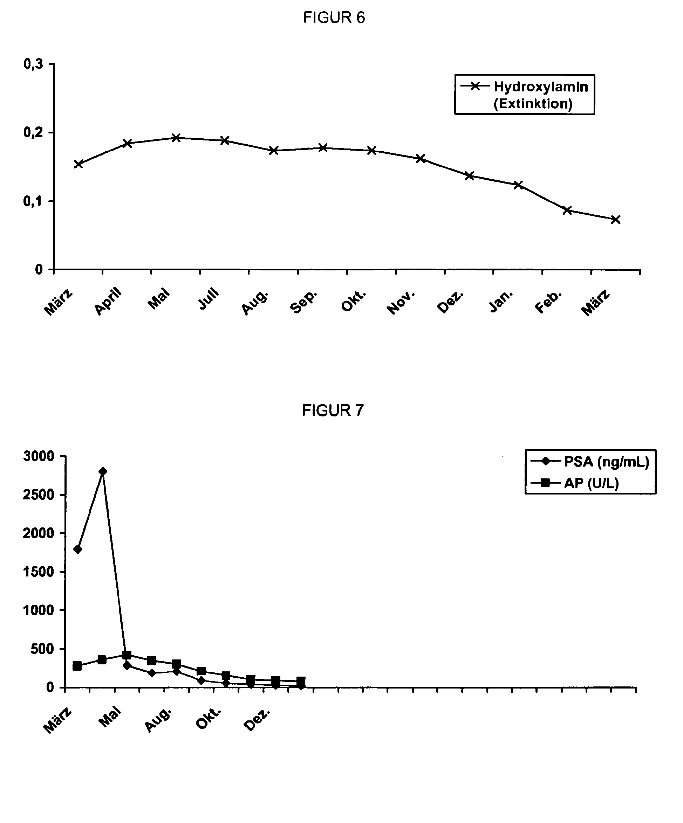

Zwei Monate nach Behandlungsbeginn wurde ein kontinuierlicher Rückgang der Tumormarker, d.h. des PSA- und des CPSA-Wertes, des CRP- und des Harnsäure-Wertes beobachtet, wobei der Hydroxylaminwert weiterhin erhöht war (Fig. 5 und Fig. 6). Der Hydroxylaminwert ging 8 Monate nach Behandlungsbeginn kontinuierlich zurück und im MRT war ein Jahr nach Behandlungsbeginn kein Tumor mehr sichtbar. Somit gab es keine weitere Indikation zur OP oder Chemotherapie mehr. In Bezug auf das Allgemeinbefinden, war der Patient während des Synoverin-Therapieverlaufs immer in guter Allgemeinverfassung. Diese besserte sich jedoch 8 Monate nach Behandlungsbeginn deutlich.

Somit führte die Behandlung mit Synoverin bei diesem Patient dazu, dass das frühproliferative Geschehen signifikant zurückgegangen ist, mit einem deutlichen Korrelationseffekt zwischen Tumormarker, Hydroxylaminwert und/oder Allgemeinbefinden. Zur Prophylaxe war eine Fortführung der Synoverin-Therapie notwendig.

### BEISPIEL 4

Bei einem Patienten, 73 Jahre alt, wurde ein ossär metastasierendes Prostatakarzinom (Adeno) mit einem Gleason-Score von 3+2=5 diagnostiziert. Die Untersuchung im Rahmen der vorliegenden Erfindung ergab folgenden Befund: Eine ausgeprägte Metastasierung in den Beckenschaufeln im Os sacrum, sowie links im Bereich des Hüftkopfes, des Schenkelhalses und des proximalen Femur. Rechtsseitig einzelne beginnende Metastasierung, auch im Hüftkopf, sowie im Schenkelhals. Eine deutlich vergrößerte Prostata mit inhomogenen Dichtewerten, eine paraaortale Lymphdrüsenvergrößerung und nicht mehr abzugrenzende Samenblasen, einen PSA-Wert von 1793; BSG-Wert von 65/99; CRP-Wert von 20,3; AP-Wert von 258; Borrelien IgG 93,2 U/ml. Das Hydroxylamin war sehr hoch, das weitere Routinelabor aber normal.

Auf ausdrücklichen Wunsch des Patienten wurde keine OP, Chemo- oder Strahlentherapie durchgeführt. Der Patient wurde bereits mit den Medikamenten Eligard^{®} (Fa. Medigene AG, Martinsried, Deutschland) (3-Monatsspritze) und Zometa^{®} (Fa. Novartis Pharma GmbH, Nürnberg, Deutschland) mit dem Wirkstoff Zoledronsäure behandelt.

Mit der Behandlung mit Synoverin wurde sofort begonnen. Über 20 Wochen wurde zweimal täglich 1 Suppositorium Synoverin und zweimal täglich 1 Kapsel Synoverin verabreicht. Im Anschluss an die 20 Wochen wurde abends 1 Suppositorium Synoverin und zweimal täglich 1 Kapsel Synoverin verabreicht. Begleitend erfolgte eine Substitution mit Mineralstoffen, Aminosäuren, Vitaminen, Enzymen und Probiotika, wobei die Organfunktionen zusätzlich durch Phytotherapie unterstützt wurden. Außerdem erfolgte eine Emährungsumstellung auf basische gluten-/lactosefreie Kost. Bei der ambulanten Behandlung wurde Synoverin über 20 Wochen täglich i.m. verabreicht, danach dreimal wöchentlich. Begleitend erfolgte eine Substitution mit Vitaminen.

Zwei Monate nach Behandlungsbeginn konnte ein kontinuierlicher Rückgang des CRP-Wertes beobachtet werden, wobei der Wert des Tumormarkers PSA und der Hydroxylaminwert ungefähr eindreiviertel Jahre nach Behandlungsbeginn kontinuierlich zurückgingen. Im CT wurde 8 Monate nach Behandlungsbeginn eine Teilremission bei ausgedehnter ossärer Metastasierung beobachtet, wobei die Herde kleiner und auch in der Nuklidintensität geringer waren. Ferner traten auch keine Herde mehr neu auf. Der Befund zeigte eine stetige Besserung und eindreiviertel Jahre nach Behandlungsbeginn war im CT eine reduzierte Tumormasse sichtbar und es traten keine neuen Rezidive auf, so dass es keine weitere Indikation zur OP oder Chemotherapie mehr gab. Im Bezug auf das Allgemeinbefinden litt der Patient zum Zeitpunkt der Anamnese unter einer atrophierten Oberschenkelmuskulatur, Gewichtsabnahme und Appetitlosigkeit. In den ersten 8 Wochen nach Behandlungsbeginn litt er unter Müdigkeit, Hautjucken und Appetitlosigkeit. Anschließend besserte sich das Allgemeinbefinden und war insgesamt in einem stabilen Zustand.

Die Behandlung mit Synoverin führte bei diesem Patient dazu, dass das proliferative Geschehen signifikant zurückgegangen ist, mit einem Korrelationseffekt zwischen Tumormarker, Hydroxylaminwert und/oder Allgemeinbefinden. Wegen der schlechten Prognose war eine Fortführung der Synoverin-Therapie erforderlich.

### BEISPIEL 5

Bei einer Patientin, 36 Jahre alt, wurde Hashimoto, ein rezidiver grippaler Infekt mit schmerzhafter Lymphknotenschwellung im anterioren oder posterioren cervicalen oder axillären Bereich, mit Muskelbeschwerden und Schmerzen in den Armen und Oberschenkeln, großer Müdigkeit, einem blassen Hauttugor, Denkschwierigkeiten, Unvermögen, sich zu konzentrieren, einer Depression, chronischer Erschöpfung und einem leichten Fieber diagnostiziert. Es gab keinen Vorbefund, aber einen Verdacht auf EBV (Epstein-Barr Virus). Das CT und MRT blieben ohne Befund und ein Karzinom wurde ausgeschlossen. Die Milz und Leber waren leicht vergrößert, wobei es für Herz und Niere keinen Befund gab. Die Patientin war arbeitsunfähig. Im Rahmen der vorliegenden Erfindung wurde herausgefunden, dass der TSH-Basal-Wert (Thyreoidea-stimulierendes Hormon) 22,1 mU/l; Anti-TPO-Wert (Autoantikörper gegen die Thyreoidale Peroxidase) 3698; EBV-VCA IGM/IgG - Wert (IgG- bzw. IgM-Antikörper gegen Antigene des Epstein-Barr Virus (EBV) in humanem Serum) erhöht und die Leukozytenanzahl gesamt 5,5 und die Lymphozyten-Subpopulation pathologisch war: CD 4-Zellen 356; CD 8-Zellen 1462; CD-3-HLA-DR+ (positiv) 650 und NK-Zellen 78. Außerdem waren die CRP-, GGT-, Bilirubin- und Hydroxylamin-Werte hoch und es wurde eine Mineralstoffwechselstörung diagnostiziert, wobei das sonstiges Labor und die Serologie ohne Befund blieb.

Mit der Behandlung ausschließlich mit Synoverin wurde sofort begonnen. Ziel der Therapie der vorliegenden Erfindung war der Immunaufbau und die Verbesserung der körperlichen Verfassung zur Herstellung der Arbeitsfähigkeit, Agilität und Symptomfreiheit. Über 20 Wochen wurde zweimal täglich 1 Suppositorium Synoverin und zweimal täglich 1 Kapsel Synoverin verabreicht. Begleitend erfolgte eine Substitution mit Mineralstoffen, Aminosäuren, Vitaminen, Enzymen und Probiotika. Außerdem wurde die Ernährung auf basische gluten-/lactosefreie Kost umgestellt. Bei der ambulanten Behandlung wurde Synoverin über 15 Wochen dreimal pro Woche i.m. verabreicht und mit Vitaminen substituiert. Sechzehn Monate nach Behandlungsbeginn folgte die Verabreichung von 1 Suppositorium Synoverin täglich und begleitend eine Substitution mit Vitaminen.

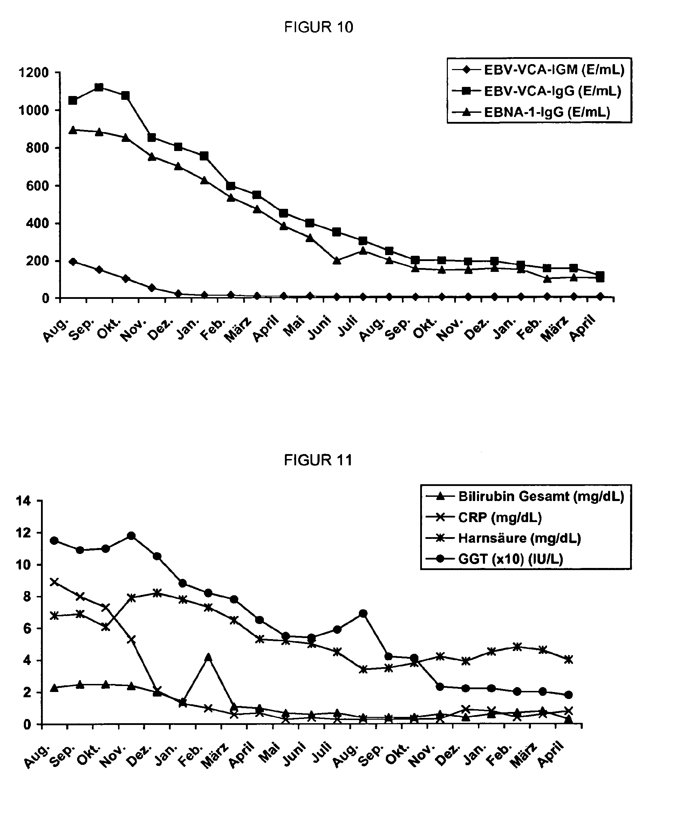

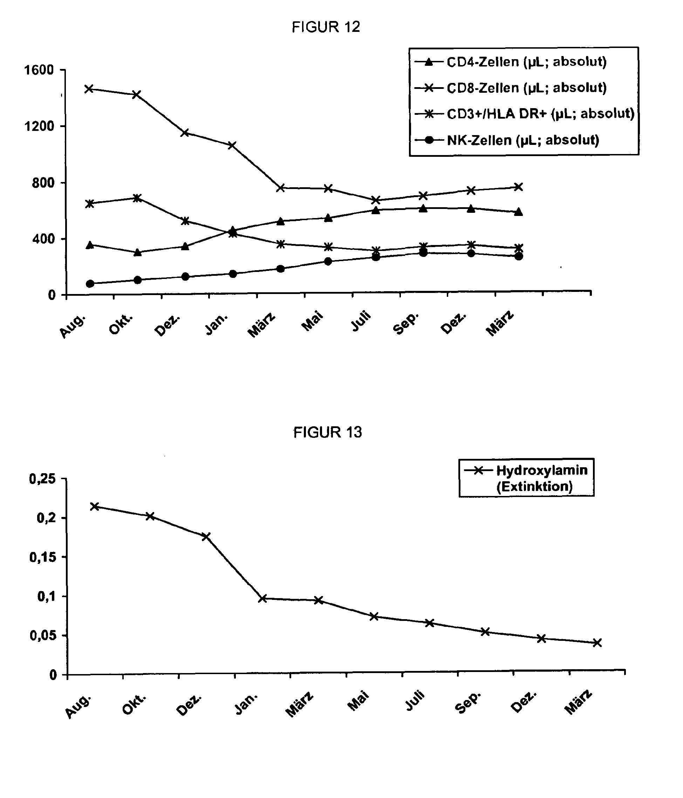

Die erhöhten Werte des Hydroxylamins, der Harnsäure, des CRP, des Bilirubins. der EBV-und EBNA-Antikörper gingen 10 Wochen nach Behandlungsbeginn kontinuierlich zurück (siehe Fig. 10-13). Bezüglich des Allgemeinbefindens hatte die Patientin zum Zeitpunkt der Anamnese depressive Phasen zur sonstigen schlechten Konstitution. Das Allgemeinbefinden besserte sich aber sofort nach Behandlungsbeginn, wobei sich nach 3 Monaten eine deutlich Besserung zeigte. Vier Monate nach Behandlungsbeginn war die Patientin erste Tage wieder am Arbeitsplatz. Sie war seitdem symptomfrei und in einem sehr guten stabilen Zustand.

Die Behandlung mit Synoverin führte bei dieser Patientin dazu, dass das rezidive Geschehen signifikant zurückgegangen ist. Auch hier zeigte sich ein deutlicher Korrelationseffekt zwischen dem Viren-Antikörper-Titer, den Entzündungswerten, der Lymphozyten-Subpopulation, dem Hydroxylaminwert und/oder dem Allgemeinbefinden. Da ein hohes Virenlast-Risiko bestand, war zur Prophylaxe eine Fortführung der Synoverin-Therapie notwendig.

### BEISPIEL 6

Ein Patient, 43 Jahre alt, war an einem Hodenkarzinom, d.h. an einem embryonalen Karzinom mit Teratom, erkrankt. Es wurden Metastasen in der Lunge und im Abdomen festgestellt und eine Orchiektomie, Strahlen- und Chemotherapie erfolgte 7 Jahre vor der Anamnese im Rahmen der vorliegenden Erfindung. Außerdem wurde ein Herpes Zoster Rezidiv (Gürtelrose), aber kein Karzinom-Rezidiv beobachtet. Die Untersuchung im Rahmen der vorliegenden Erfindung ergab den folgenden Befund: Eine ausgeprägte Gürtelrose entlang der Brustwirbel und Rippen (beidseitig), Nervenschmerzen an den Füßen und Beinen, Lidzucken am rechten Auge, Hepatitis mit leichtem Ikterus, ein geblähtes Abdomen ohne Aszites, Appetitlosigkeit, Lethargie, Kraftlosigkeit und eine Depression. Der Herpes Zoster Antikörper IgG-Wert war 3214 und die Lymphozyten-Subpopulation pathologisch. Dies ergab einen Immundefekt, wobei die Harnsäure-, CRP-, GGT-, Bilirubin- und Hydroxylamin-Werte hoch waren. Das sonstige Labor blieb ohne Befund.

Mit der Behandlung ausschließlich mit Synoverin wurde sofort begonnen. Über 20 Wochen wurde zweimal täglich 1 Suppositorium Synoverin und zweimal täglich 1 Kapsel Synoverin verabreicht. Begleitend erfolgte eine Substitution mit Mineralstoffen, Aminosäuren, Vitaminen, Enzymen und Probiotika. Außerdem erfolgte eine Ernährungsumstellung auf basische gluten-/lactosefreie Kost und die Organfunktionen wurden durch Phytotherapie unterstützt. Bei der ambulanten Behandlung über 6 Wochen wurde täglich und danach dreimal pro Woche Synoverin i.m. verabreicht. Begleitend erfolgten Vitamin-Substitutionen. Fünf Monate nach Behandlungsbeginn folgte die Verabreichung von 1 Suppositorium Synoverin täglich, einmal wöchentlich die Verabreichung von Synoverin i.m. mit begleitender Vitaminverabreichung.

Drei Monate nach Behandlungsbeginn konnte ein kontinuierlicher Rückgang der erhöhten Herpes Zoster Antikörper- (Herpes Zoster IgG), Hydroxylamin-, Harnsäure-, Bilirubin- und CRP-Werte und außerdem eine Verbesserung der Symptomatik und ein Rückgang des Ikterus beobachtet werden. Vier Monate nach Behandlungsbeginn wurde keine Hepatitis mehr beobachtet und der Befund zeigte eine stetige Besserung. Nach 9 Monaten waren alle Laborwerte im Normbereich und es traten keine Rezidiven mehr auf. Im Bezug auf das Allgemeinbefinden litt der Patient zum Zeitpunkt der Anamnese unter starken Schmerzen, Schwächeanfällen, einer Gewichtsabnahme und Appetitlosigkeit, wobei sich das Allgemeinbefinden 10 Tage nach Behandlungsbeginn mit Synoverin deutlich besserte. Der Patient konnte 2 Monate nach Behandlungsbeginn seiner Arbeit wieder im vollen Umfang nachgehen und war nach 9 Monaten symptomfrei und in einem sehr guten stabilen Zustand.

Die Behandlung mit Synoverin führte bei diesem Patient dazu, dass das rezidive Geschehen signifikant zurückgegangen ist. Außerdem wurde ein deutlicher Korrelationseffekt zwischen Herpes Antikörpern, Entzündungswerten, Lymphozyten-Subpopulation, Hydroxylaminwert und/oder Allgemeinbefinden beobachtet. Da ein hohes Rezidiv-Risiko bestand, war zur Prophylaxe eine Fortführung der Synoverin-Therapie notwendig.

### BEISPIEL 7

Ein Patient, 43 Jahre alt, zeigte bei der Anamnese rezidive grippale Infekte mit Bronchitis, eine Darm-Candidose, große Müdigkeit, einen blassen Hauttugor, hatte aber keinen Vorbefund. Es bestand ein Verdacht auf HIV. CT und MRT blieben ohne Befund und ein Karzinom wurde ausgeschlossen. Außerdem blieben Abdomen, Herz, Niere und Leber ohne Befund. Die Untersuchung im Rahmen der vorliegenden Erfindung ergab, dass der Patient HIV-positiv war, mit einer Virenlast von 6940 Kopien, einer Leukozytenanzahl (gesamt) von 5,3 und einer pathologischen Lymphozyten-Subpopulation: CD 4-Zellen 556, CD 8-Zellen 1062, CD-3-HLA-DR-positiv (+) 500 und NK-Zellen 81. Es wurde eine generalisierte Candidose bei einem Körpergewicht von 78 kg und einer Größe von 178 cm beobachtet. Außerdem hatte der Patient ein geblähtes Abdomen ohne Aszites und war depressiv, so dass insgesamt auf einen Immundefekt geschlossen wurde. Die Harnsäure-, CRP-, GGT-, Bilirubin- und Hydroxylamin-Werte waren hoch, wobei das sonstiges Labor ohne Befund blieb. Die Serologie ergab eine Cytomegalie, Toxoplasmose und eine abgelaufene Hepatitis B. Ansonsten war der Patient aber ohne Befund.

Mit der Behandlung mit Synoverin wurde sofort begonnen. Ziel dieser Therapie war der Immunaufbau, die Verbesserung der körperlichen Verfassung, die Beseitigung der Candidose und ein möglichst langer Zeitraum ohne HAART-Therapie. Über 20 Wochen wurde einmal täglich 1 Suppositorium Synoverin und zweimal täglich 1 Kapsel Synoverin verabreicht. Begleitend erfolgte eine Substitution mit Mineralstoffen, Aminosäuren, Vitaminen, Enzymen, diätetischen Lebensmitteln und Probiotika. Ferner wurde die Organfunktionen durch Phytotherapie unterstützt und die Ernährung auf basische gluten-/lactosefreie Kost umgestellt. Bei der ambulanten Behandlung mit dreimal pro Woche Synoverin (i.m.) über 15 Wochen, wurden begleitend Vitamine verabreicht. Fünf Monate nach Behandlungsbeginn folgte die Verabreichung von 1 Suppositorium Synoverin täglich, einmal wöchentlich Synoverin i.m. und begleitend eine Vitamin-Verabreichung.

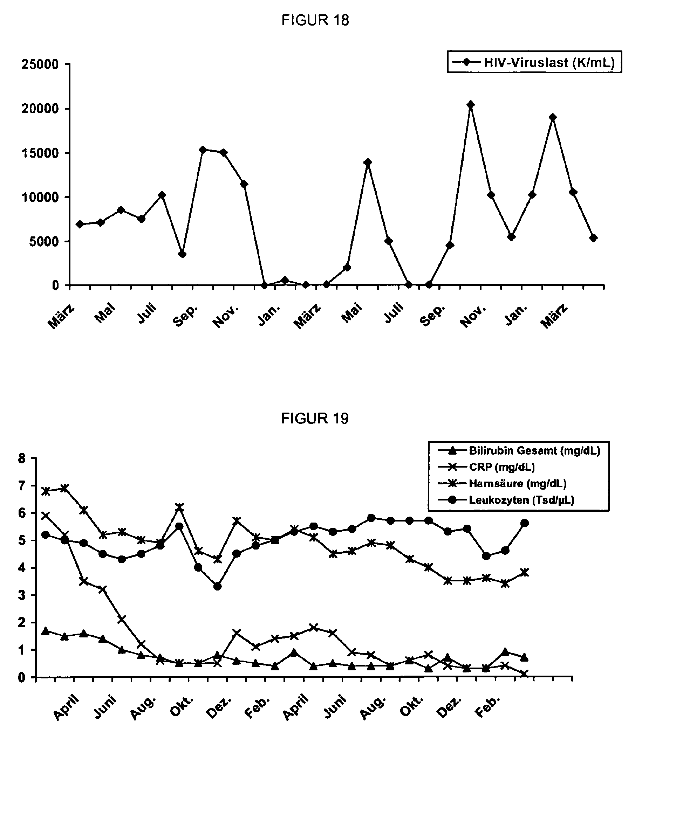

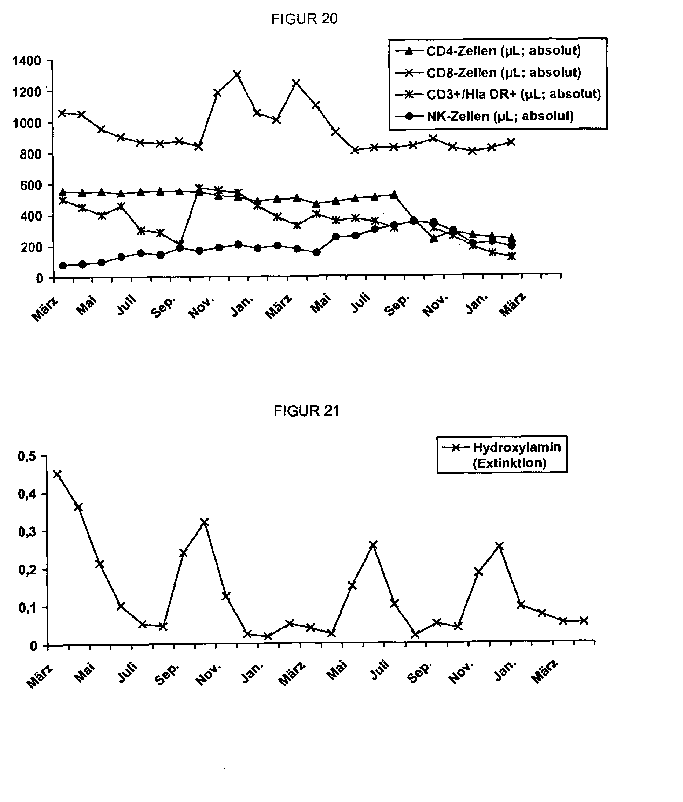

Über 6 Wochen nach Behandlungsbeginn wurde eine Reduktion der Virenlast, des Hydroxylamins, der Harnsäure und des CRP beobachtet (siehe Fig. 18-21). Elf Monate nach Behandlungsbeginn ergab sich ein kontinuierlicher Rückgang dieser erhöhten Werte, wobei nach 14, 19 und 23 Monaten die Virenlast rasant anstieg. Hier wurde sofort jeweils intensiver therapiert, so dass die Virenlast wieder sank. Das Allgemeinbefinden verbesserte sich sofort nach Behandlungsbeginn deutlich. Insgesamt war der Patient auch noch zwei Jahre nach Behandlungsbeginn symptomfrei und in einem sehr guten stabilen Zustand.

Die Behandlung mit Synoverin führte bei diesem Patient zu einem signifikanten Rückgang des rezidiven Geschehens, zu einer raschen Verbesserung des Allgemeinbefindens und zu einer Reduktion der Virenlast. Zwischen Virenlast, Entzündungswerten, Lymphozyten-Subpopulation und Hydroxylaminwert gab es einen deutlichen Korrelationseffekt. Außerdem korrelierte die Virenlast mit dem Allgemeinbefinden des Patienten. Da ein hohes Virenlast-Risiko bestand, war zur Prophylaxe eine Fortführung der Synoverin-Therapie notwendig, um eine lange Zeitspanne bis zum Einsatz von HAART zu erreichen.

### BEISPIEL 8

Ein Patient, 41 Jahre alt, zeigte zum Zeitpunkt der Anamnese eine starke Gewichtsabnahme, große Müdigkeit, hatte aber ansonsten keinen Befund. Es bestand ein Verdacht auf HIV. CT und MRT blieben ohne Befund, so dass ein Karzinom ausgeschlossen worden war. Die Untersuchung im Rahmen der vorliegenden Erfindung ergab, dass der Patient HIV-positiv war, mit einer Virenlast von 5 006 000 Kopien, einer Leukozytenanzahl gesamt von 1,9 und einer pathologischen Lymphozyten-Subpopulation: CD 4-Zellen 78, CD 8-Zellen 346, CD-3-HLA-DR-positiv (+) 79 und NK-Zellen 78. Es wurde eine generalisierte Candidose bei einem Körpergewicht von 48 kg bei einer Größe von 178 cm beobachtet. Der Patient war nicht arbeitsfähig und hatte ein geblähtes Abdomen ohne Aszites. Er klagte außerdem über Appetitlosigkeit, war lethargisch, kraftlos, aber nicht depressiv. Es wurde eine Pruritis am ganzen Körper diagnostiziert, so dass insgesamt auf einen Immundefekt geschlossen wurde. Die Harnsäure-, CRP-, GGT-, Bilirubin-, Hydroxylamin-Werte waren hoch, wobei das sonstige Labor aber ohne Befund war. Die Serologie ergab, dass die Chlamydien pneum. und Mykoplasmen-Werte leicht erhöht waren. Allerdings litt er nicht an einer Hepatitis und war auch sonst ohne Befund.

Ziel der Therapie der vorliegenden Erfindung war der Immunaufbau, die Verbesserung der körperlichen Verfassung, eine Gewichtszunahme, die Beseitigung der Candidose als Vorbereitung auf das HAART-Programm. Bis dahin wurde auf ausdrücklichen Wunsch des Patienten keine Chemotherapie durchgeführt. Die Behandlung ausschließlich mit Synoverin begann sofort. Über 20 Wochen wurde einmal täglich 1 Suppositorium Synoverin und zweimal täglich 1 Kapsel Synoverin verabreicht. Begleitend erfolgte eine Substitution mit Mineralstoffen, Aminosäuren, Vitaminen, Enzymen, diätetischen Lebensmitteln und Nystatin und die Ernährung wurde auf hochkalorische, basische gluten/lactosefreie Kost umgestellt. Danach erfolgte die Substitution von Probiotika und die Organfunktionen wurden durch Phytotherapie unterstützt. Bei der ambulanten Behandlung über sechs Wochen wurde Synoverin i.m. täglich und danach dreimal pro Woche verabreicht und begleitend erfolgten Vitamin-Substitutionen. Zehn Monate nach Behandlungsbeginn folgte die Verabreichung von 1 Suppositorium Synoverin täglich, einmal wöchentlich Synoverin i.m. und begleitend erfolgten Vitamin- Substitutionen.

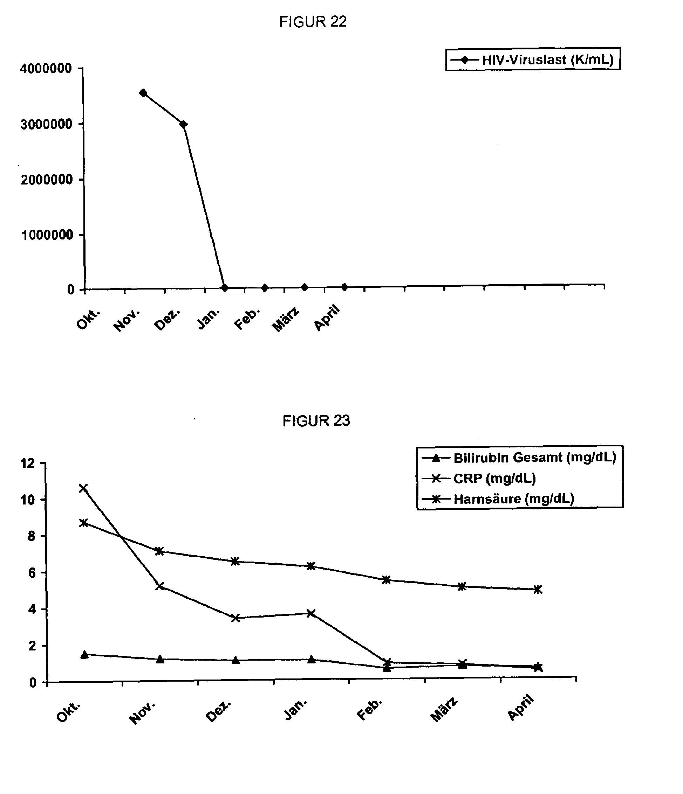

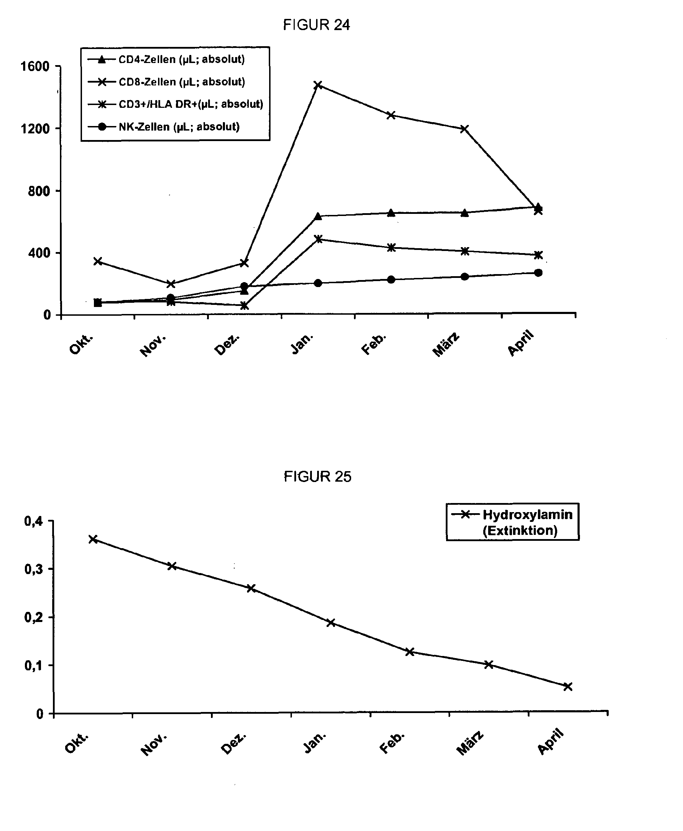

Über 6 Wochen nach Behandlungsbeginn zeigte sich eine sofortige, deutliche Verbesserung des Allgemeinbefindens. Außerdem wurde eine Reduktion der Virenlast, des Hydroxylamins, der Harnsäure und des CRP beobachtet (siehe Fig. 22-25). Nach 10 Tagen war der Patient wieder arbeitsfähig und es wurde eine Gewichtszunahme beobachtet. Außerdem wurde eine Verbesserung der Symptomatik, ein Rückgang von Pruritis und Gichtanfällen beobachtet und es trat kein Ikterus auf. Zwei Monate nach Behandlungsbeginn startete das HAART-Programm. Er nahm stetig an Gewicht zu und war vollumfänglich arbeitsfähig. Der Befund zeigte stetige Besserung. Vier Monate nach Behandlungsbeginn waren alle Laborwerte im Normbereich und die HIV-Viruslast < 50 Kopien. Auch ein halbes Jahr nach Behandlungsbeginn zeigte der Patient keine neuen Rezidive.

Die Behandlung mit Synoverin führte bei diesem Patient dazu, dass das rezidive Geschehen signifikant zurückgegangen ist. Durch die Vortherapie war die Verträglichkeit des HAART-Programms deutlich verbessert. Die Virenlast wurde rasch reduziert und es gab einen deutlichen Korrelationseffekt zwischen Virenlast, Entzündungswerten, Lymphozyten-Subpopulation, Hydroxylaminwert und/oder Allgemeinbefinden. Da ein hohes Rezidiv-Risiko bestand, war zur Prophylaxe eine Fortführung der Synoverin-Therapie notwendig.

### BEISPIEL 9

Ein Patient, 25 Jahre alt, zeigte zum Zeitpunkt der Anamnese rezidive Infekte mit produktiver Sinusitis und Bronchitis, leichtes Fieber, Darmdysbiose, Gewichtsverlust (Reduktion der Muskelmasse), große Müdigkeit, blassen Hauttugor und eine drastische Abnahme der Leistungsfähigkeit. Außerdem klagte er über Muskelschmerzen, wobei es keinen Vorbefund gab. Das CT und MRT blieben ohne Befund, so dass ein Karzinom ausgeschlossen worden war. Zudem blieben Abdomen, Herz, Niere, Leber ohne Befund und das EKG war normal. Die Untersuchung im Rahmen der vorliegenden Erfindung ergab eine Leukozytenanzahl gesamt von 9,8 und eine pathologische Lymphozyten-Subpopulation: CD 4-Zellen 421, CD 8-Zellen 1062, CD-3-HLA-DR-pos. 500, NK-Zellen 69. Außerdem wurde eine Darm-Candidose bei einem Körpergewicht von 80 kg und einer Größe von 192 cm beobachtet. Ferner war das Abdomen ohne Aszites gebläht und die IgM-, IgA-, Harnsäure-, CRP-, CPK-, Bilirubin-, Hydroxylamin-Werte waren hoch. Das sonstige Labor und die Serologie blieben ohne Befund.

Ziel der Therapie der vorliegenden Erfindung war der Immunaufbau, die Verbesserung der körperlichen Verfassung, der Aufbau der Muskelmasse, die Beseitigung der Candidose in möglichst kurzer Zeit. Die Behandlung ausschließlich mit Synoverin begann sofort. Über 20 Wochen wurde einmal täglich 1 Suppositorium Synoverin und dreimal täglich 1 Kapsel Synoverin verabreicht. Begleitend erfolgte eine Substitution mit Mineralstoffen, Aminosäuren, Vitaminen, Enzymen, diätetischen Lebensmitteln und Probiotika. Ferner wurde die Ernährung auf basische gluten/lactosefreie Kost umgestellt und die Organfunktionen durch eine Entschlackung unterstützt. Fünf Monate nach Behandlungsbeginn folgte die Verabreichung von dreimal täglich 1 Kapsel Synoverin und begleitend die Verabreichung von Vitaminen.

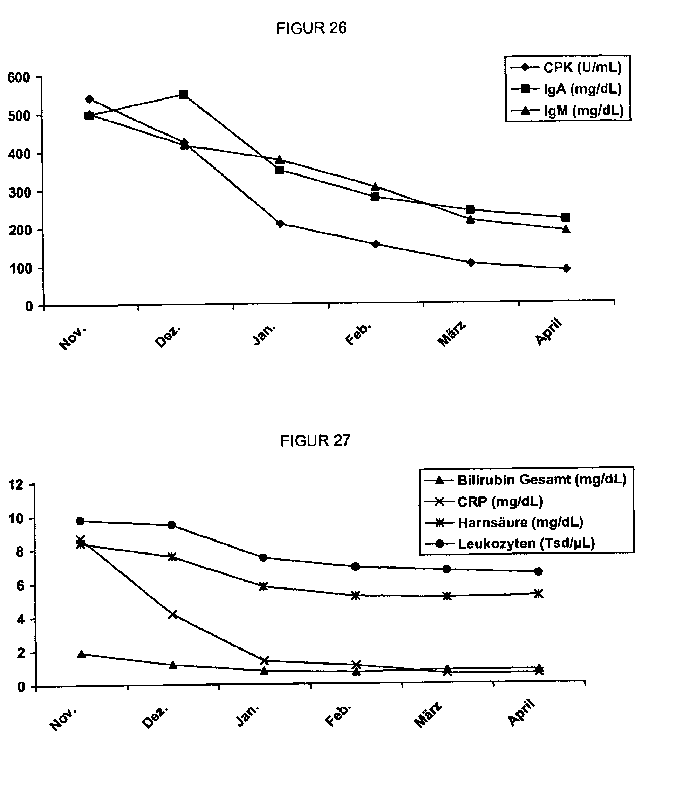

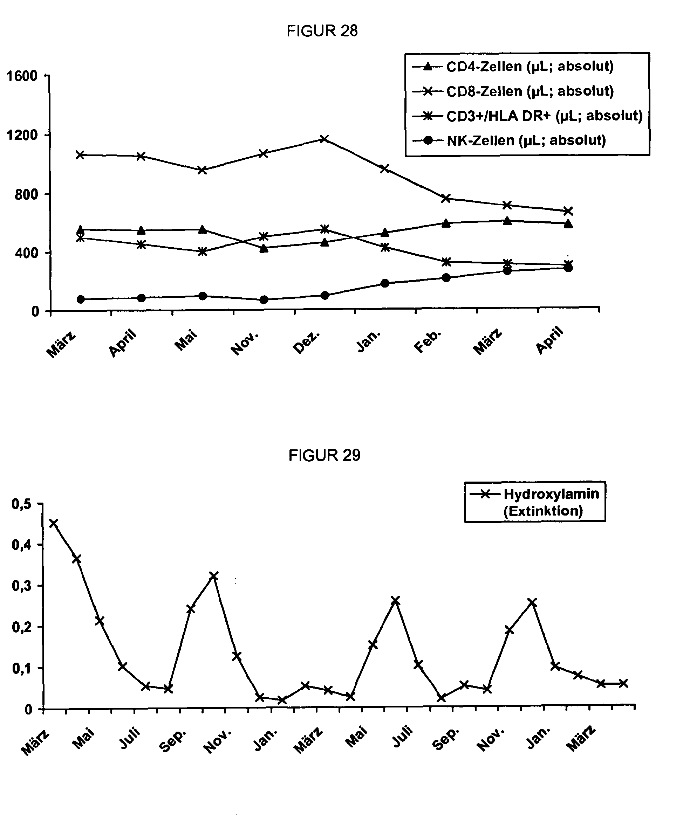

Es wurde ein deutlicher Korrelationseffekt zwischen Entzündungswerten, Lymphozyten-Subpopulation, Hydroxylaminwert und/oder dem Allgemeinbefinden beobachtet (siehe Fig. 26-29). Insgesamt war er 4 Monate nach Behandlungsbeginn symptomfrei und in einem sehr guten stabilen Leistungszustand. Die Behandlung mit Synoverin führte bei diesem Patient somit zu einem signifikanten Rückgang einer beginnenden Immunschwäche und eines rezidiven Geschehens und zu einer raschen Verbesserung des Allgemeinbefindens. Somit zeigte Synoverin eine sehr gute Wirkung auf das beginnende Immungeschehen und verkürzte die Regenerationszeit bei Überbeanspruchung der Muskulatur. Zur Prophylaxe war eine Fortführung der Synoverin-Therapie notwendig.

## Patentansprüche

1. Pharmazeutische Zusammensetzung enthaltend die Wirkstoffkombination aus 1-(2-Diethylaminoethyl)-3-(*p*-methoxy-benzyl)-1,2-dihydrochinoxalin-2-on und 7β-Hydroxycholesterol oder dessen pharmazeutisch verträgliche Salze ausgewählt aus 7β-Hydroxycholesterol-bis-hemsuccinat-di-natriumsalz oder 7β-Hydroxycholesterol-bis-hemsuccinat-diethanolaminoat zur Verwendung bei der Erhöhung der Leistungsfähigkeit des Immunsystems.

2. Pharmazeutische Zusammensetzung zur Verwendung gemäß Anspruch 1, wobei die Immunleistungsfähigkeit bei Krebs, bakteriellen oder viralen Erkrankungen, Autoimmunerkrankungen oder erhöhter Stress- oder Umweltbelastung erhöht wird.

3. Pharmazeutische Zusammensetzung zur Verwendung gemäß Anspruch 1 oder 2, wobei bei viralen Erkrankungen die Virenlast ohne die Gabe von hochaktiver antiretroviraler Therapie und/oder Interferon gesenkt wird.

4. Pharmazeutische Zusammensetzung zur Verwendung gemäß Anspruch 3, wobei die viralen Erkrankungen vom HI-, Hepatitis-A-, Hepatitis-B-, Hepatitis-C-, Varizella-Zoster-, Epstein-Barr- oder Cytomegalie-Virus hervorgerufen werden.

5. Pharmazeutische Zusammensetzung zur Verwendung gemäß Anspruch 1 oder 2, wobei bei Autoimmunerkrankungen die chronische Entzündung gesenkt wird.

6. Pharmazeutische Zusammensetzung zur Verwendung gemäß Anspruch 5, wobei die Autoimmunerkrankung aus der Gruppe ausgewählt wird, die aus Hashimoto-Thyreoiditis, Morbus Basedow, Morbus Crohn, Rheuma, Psoriasis, Fibromyalgie oder Multipler Sklerose besteht.

7. Pharmazeutische Zusammensetzung zur Verwendung gemäß Anspruch 1, wobei bei erhöhter Stressbelastung die körpereigene immunaktivität, die Anwahl der NK-Zellen und Leukozyten erhöht wird.

8. Pharmazeutische Zusammensetzung zur Verwendung gemäß einem der Ansprüche 1-7, wobei die Wirkstoffkombination 20-200 mg 1-(2-Diethylaminoethyl)-3-(*p-*methoxy-benzyl)-1,2-dihydrochinoxalin-2-on und 5-200 mg des 7β-Hydroxycholesterols enthält.

9. Pharmazeutische Zusammensetzung zur Verwendung gemäß einem der Ansprüche 1-8, wobei die Wirkstoffkombination oral, parenteral, kutan, subkutan, intravenös, intramuskulär oder rektal zu verabreichen ist.

10. Pharmazeutische Zusammensetzung zur Verwendung gemäß einem der Ansprüche 1-9, wobei die Wirkstoffkombination als Lösung getrennt in zwei Ampullen enthalten ist.

11. Pharmazeutische Zusammensetzung zur Verwendung gemäß einem der Ansprüche 1-10, wobei die Wirkstoffkombination als Lösung in einer Ampulle, als Kapsel, Salbe oder Suppositorium zu verabreichen ist.

12. Pharmazeutische Zusammensetzung zur Verwendung gemäß einem der Ansprüche 1-11 wobei die Wirkstoffkombination 1-7 Mal pro Woche, vorzugsweise 3 Mal pro Woche, eine oder zwei Ampullen per Injektion, und/oder 1-7 Mal pro Woche, vorzugsweise täglich, vorzugsweise abends, ein Suppositorium und/oder 1-14 Mal pro Woche, vorzugsweise 2 Mal täglich, vorzugsweise morgens und abends, eine Hartkapsel zu verabreichen ist.

13. Pharmazeutische Zusammensetzung zur Verwendung gemäß einem der Ansprüche 1-12, wobei von der Wirkstoffkombination dreimal wöchentlich eine Ampulle und jeden Abend ein Suppositorium, und/oder zweimal täglich eine Hartkapsel, jeweils morgens und abends, zu verabreichen ist.

## Claims

1. A pharmaceutical composition containing the active substance combination of 1-(2-diethylaminoethyl)-3-(p-methoxy-benzyl)-1,2-dihydroquinoxalin-2-one and 7ß-hydroxycholesterol or the pharmaceutically compatible salts thereof, selected from 7β-hydroxycholesterol-bis-hemsuccinate-disodium salt or 7β-hydroxycholesterol-bis-hemsuccinate-diethanolaminoate for use in increasing the performance of the immune system.

2. The pharmaceutical composition for use according to claim 1,
wherein the immune performance is increased in the case of cancer, bacterial or viral diseases, autoimmune diseases or increased stress or environmental impact.

3. The pharmaceutical composition for use according to claim 1 or 2, wherein in the case of viral diseases the viral load is lowered without the administration of highly active antiretroviral therapy and/or interferon.

4. The pharmaceutical composition for use according to claim 3,
wherein the viral diseases are caused by the HI, hepatitis A, hepatitis B, hepatitis C, varicella zoster, Epstein-Barr virus or cytomegalovirus.

5. The pharmaceutical composition for use according to claim 1 or 2, wherein a chronic inflammation is lowered in the case of autoimmune diseases.

6. The pharmaceutical composition for use according to claim 5,
wherein the autoimmune disease is selected from the group consisting of Hashimoto's thyroiditis, Graves' disease, Crohn's disease, rheumatism, psoriasis, fibromyalgia or multiple sclerosis.

7. The pharmaceutical composition for use according to claim 1,
wherein in the case of increased stress the body's own immune activity, the numbers of NK cells and leukocytes are increased.

8. The pharmaceutical composition for use according to any of claims 1 to 7, wherein the active substance combination contains 20 - 200 mg 1-(2-diethylaminoethyl)-3-(p-methoxy-benzyl)-1,2-dihydroquinoxalin-2-one and 5 - 200 mg 7β-hydroxycholesterol.

9. The pharmaceutical composition for use according to any of claims 1 to 8, wherein the active substance combination is to be administered orally, parenterally, cutaneously, subcutaneously, intravenously, intramuscularly or rectally.

10. The pharmaceutical composition for use according to any of claims 1 to 9, wherein the active substance combination is contained separately in two ampoules as a solution.

11. The pharmaceutical composition for use according to any of claims 1 to 10, wherein the active substance combination is to be administered as a solution in an ampoule, as a capsule, ointment or suppository.

12. The pharmaceutical composition for use according to any of claims 1 to 11, wherein the active substance combination is to be administered as follows: one or two ampoules per injection 1 to 7 times a week, preferably 3 times a week, and/or a suppository 1 to 7 times a week, preferably daily, preferably in the evening, and/or a hard capsule 1 to 14 times a week, preferably twice daily, preferably in the morning and evening.

13. The pharmaceutical composition for use according to any of claims 1 to 12, wherein the active substance combination is to be administered as follows: an ampoule three times a week and a suppository every evening and/or a hard capsule twice daily, i.e. in the morning and evening.

## Revendications

1. Composition pharmaceutique contenant la combinaison de substances actives de 1-(2-diéthylaminoéthyl)-3-(p-méthoxy-benzyl)-1,2-dihydrochinoxalin-2-one et 7β-hydroxycholestérol ou leurs sels acceptables pharmaceutiquement choisis parmi 7β-hydroxycholestérol-bis-hemsuccinat-disodium ou 7β-hydroxycholestérol-bis-hemsuccinat-diéthanolaminoate destinée à être utilisée pour augmenter la capacité du système immunitaire.

2. Composition pharmaceutique destinée à être utilisée selon la revendication 1, dans laquelle la capacité immunitaire est augmentée lors de cancers, de maladies bactériennes ou virales, de maladies auto-immunes ou d'effets de stress ou de pollution environnementale augmentés.

3. Composition pharmaceutique destinée à être utilisée selon la revendication 1 ou 2, dans laquelle, lors de maladies virales, la charge de virus est diminuée sans l'administration d'une thérapie antirétrovirale hautement active et/ou d'interféron.

4. Composition pharmaceutique destinée à être utilisée selon la revendication 3, dans laquelle les maladies virales sont provoquées par le virus de H1, hépatite A, hépatite B, hépatite C, varicelle-zona, d'Epstein-Barr ou de cytomégalie.

5. Composition pharmaceutique destinée à être utilisée selon la revendication 1 ou 2, dans laquelle, lors de maladies auto-immunes, l'inflammation chronique peut être diminuée.

6. Composition pharmaceutique destinée à être utilisée selon la revendication 5, dans laquelle la maladie auto-immune est sélectionnée parmi le groupe composé de la thyroïdite de Hashimoto, de la maladie de Basedow, de la maladie de Crohn, du rhumatisme, de la psoriasis, de la fibromyalgie, de la sclérose multiple.

7. Composition pharmaceutique destinée à être utilisée selon la revendication 1, dans laquelle, en cas d'effets de stress augmentés, l'activité propre au corps, le nombre de lymphocytes NK et de leucocytes sont augmentés.

8. Composition pharmaceutique destinée à être utilisée selon l'une des revendications 1 à 7, dans laquelle la combinaison de substances actives contient de 20 à 200 mg de 1-(2-diéthylaminoéthyl)-3-(p-méthoxy-benzyl)-1,2-dihydrochinoxalin-2-one et de 5 à 200 mg de 7β-hydroxycholestérol.

9. Composition pharmaceutique destinée à être utilisée selon l'une des revendications 1 à 8, dans laquelle la combinaison de substances actives doit être administrée par voie orale, parentérale, cutanée, sous-cutanée, intraveineuse, intramusculaire ou rectale.

10. Composition pharmaceutique destinée à être utilisée selon l'une des revendications 1 à 9, dans laquelle la combinaison de substances actives est contenue sous forme de solution séparément dans deux ampoules.

11. Composition pharmaceutique destinée à être utilisée selon l'une des revendications 1 à 10, dans laquelle la combinaison de substances actives doit être administrée sous forme de solution dans une ampoule, de capsule, de pommade ou de suppositoire.

12. Composition pharmaceutique destinée à être utilisée selon l'une des revendications 1 à 11, dans laquelle la combinaison de substances actives doit être administrée de 1 à 7 fois par semaine, de préférence 3 fois par semaine, une ou deux ampoules par injection, et/ou 1 à 7 fois par semaine, de préférence chaque jour, de préférence le soir, un suppositoire et/ou de 1 à 14 fois par semaine, de préférence 2 fois par jour, de préférence le matin et le soir, une capsule dure.

13. Composition pharmaceutique destinée à être utilisée selon l'une des revendications 1 à 12, dans laquelle doivent être administrés trois fois par semaine une ampoule et chaque soir un suppositoire, et/ou deux fois par semaine une capsule dure, chaque fois le matin et le soir.
